# EUROPEAN PATENT APPLICATION

(11) **EP 2 614 823 A1**
(43) Date of publication of application: **17.07.2013**
(21) Application number: 12150762.8
(22) Date of filing: 11.01.2012
(51) Int. Cl.: A61K 31/4192, A61K 31/454, A61P 9/12

(54) **Use of triazolylpiperidine derivatives**

(71) Applicant: Bergen Teknologioverføring AS, 5006 Bergen (NO)
(72) Inventor: Haug, Bengt Erik, 5114 Tertnes (NO); Sydnes, Leiv K., 5239 Radal (NO); Harmsen, Rianne, 5032 Bergen (NO); Brandsdal, Bjørn Olav, 9037 Tromsø (NO)
(74) Representative: Kröncke, Rolf

(57) **Abstract**

The present invention relates to triazolylpiperidine compounds useful for the treatment of hypertension and diseases, disorders or conditions due to or involving hypertension and high blood pressure. In particular, the present invention relates to substituted triazolylpiperidine compounds representing inhibitors of aspartic proteases, in particular, of renin. Further, the present invention relates to pharmaceutical compositions containing the same useful in the treatment or prophylaxis of hypertension, preferably of essential hypertension.

## Description

The present invention relates to triazolylpiperidine compounds useful for the treatment of hypertension and diseases, disorders or conditions due to or involving hypertension and high blood pressure. In particular, the present invention relates to substituted triazolylpiperidine compounds representing inhibitors of aspartic proteases, in particular, of renin. Further, the present invention relates to pharmaceutical compositions containing the same useful in the treatment or prophylaxis of hypertension, preferably of essential hypertension.

### Prior art

Hypertension refers to a pathological condition characterized by elevated systemic blood pressure representing a major risk factor for the development of cardiovascular diseases. It is assumed that one billion people worldwide are affected with hypertension, the most of them suffer of essential (primary) hypertension (Yokokawa, F.; Maibaum, J., Expert Opin. Ther. Patents 2008, 18, 581-602). Currently, less than 30% of patients affected with this condition achieve a sufficient reduction of blood pressure and reach the individual treatment goals. This is mainly due to the less than satisfactory pharmaceutical agents currently available. Thus, a growing need exists for new classes of antihypertensive drugs with novel action mechanisms (Kearney, P.M. et al; Lancet 2005, 365, 217-223). The blood pressure is tightly regulated by multiple pathways. The renin-angiotensin system (RAS) regulates blood pressure and fluid balance by secreting renin - an enzyme belonging to the family of aspartic proteases - from the juxtaglomerular cells of the kidney into the circulating system leading to the conversion of angiotensinogen - which is secreted by the liver - to the non-active angiotensin I. The subsequent conversion of angiotensin I to angiotensin II - representing the potent vasoactive peptide triggering the constriction of blood vessels and therefore elevating the blood pressure - is effected by the lung's angiotensin converting enzyme (ACE). Furthermore, angiotensin II triggers the secretion of aldosteron, a mineralocorticoid hormone, increasing the resorption of sodium and water from the kidney's tubules and leading to increased body fluids and blood pressure. The rate limiting step of this reaction is the conversion of angiotensinogen to angiotensin I (The Renin-Angiotensin-System; Robertson, J., I., S., Nicholls, M. G., Eds.; Mosby,: London, 1993, 2 vols). Renin is therefore a key enzyme in mediating blood pressure by the RAS.

Monoclonal antibodies against renin are not suitable as pharmaceuticals as they have to be administered parenterally and most of them are highly immunogenic. Statins as transition state analogs were synthesized but exhibit only a low bioaviabilty after oral administration due to their peptide like nature (Lunney, E.A.; Humblet, C. , Medical Chemistry of the Renin-Angiotensin System, Timmermans, P.B.; Wexler, R.R. Eds.; Elsevier: New York, 1994; 21, pages 73-102). Modifications of the statins led to a compound named CGP38560 having a reduced peptidic character and lower molecular weight, which was further improved by Novartis and culminated in the development of Aliskiren- currently the only direct renin inhibitor approved by the European Medicines Agency (EMEA) and the US Food and Drug Administration (FDA) as antihypertensive drug (Jensen, C. et. al., Nature Reviews Drug Discovery, May 2008, Vol. 7, pages 399-410).

Thus, there is still an ongoing need for drugs in the treatment of hypertension, particularly in essential hypertension.

Hence, the object of the present invention is to provide new molecules inhibiting aspartic proteases like the rate determining enzyme renin, and thus, providing useful pharmaceutical compounds for the treatment of hypertension, particularly of essential hypertension as well as diseases, disorders or conditions due to or involving hypertension.

### Brief description of the present invention

In a first aspect, the present invention relates to triazolylpiperidine compounds of general formula III or IV 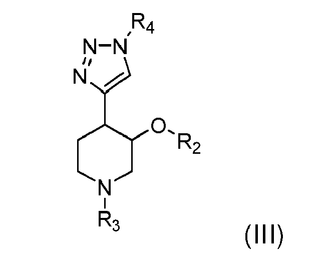 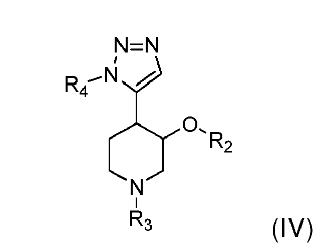 wherein R2 and R3 are independently from each other hydrogen or a C1 -C18 alkyl group, a C2 - C18 alkenyl group, a C3 - C18 aryl group, a C3 - C18 heteroalkyl group, a C3 - C18 heteroaryl group, an aralkyl group, an arylalkyl group, an aryl group, an heteroarylalkyl group, an alkoxy group, an acyl group, like a carbonylalkyl group or a carbonylaryl group, all of said groups may be substituted with at least one substituent independently selected from the group of C1-C6 alkyl, C2-C6 alkenyl, halogen, CN, NO2, C1-C6 alkoxy, hydroxyl, carbamoyl, C1 to C8 alkylcarbamoyl, amido, C1-C6 alkoxycarbonyl, C1-C6 alkylcarbamoyloxy, C1-C6 alkoxycarbonylamino, amino, C1-C6 alkanoyloxy group, C1-C6 alkylthio and sulphur-containing analogs of oxygen containing substituents,
R4 is selected from hydrogen or a C1 -C18 alkyl group, a C2 - C18 alkenyl group, a C3 - C18 aryl group, a C3 - C18 heteroalkyl group, a C3 - C18 heteroaryl group, an aralkyl group, an arylalkyl group, an aryl group, an heteroarylalkyl group, an alkoxy group, an acyl group, like a carbonylalkyl group or a carbonylaryl group, all of said groups may be substituted with at least one substituent independently selected from the group of C1-C6 alkyl, C2-C6 alkenyl, halogen, CN, NO2, C1-C6 alkoxy, hydroxyl, carbamoyl, C1 to C8 alkylcarbamoyl, amido, N-substituted amido, C1-C6 alkoxycarbonyl, C1-C6 alkylcarbamoyloxy, C1-C6 alkoxycarbonylamino, amino, C1-C6 alkanoyloxy group, C1-C6 alkylthio and sulphur-containing analogs of oxygen containing substituents, or a substituent of formula V

-X-Y-Z (V)

wherein X and Z are independently selected from a C₁ - C₁₈ alkyl group, a C₂ - C₁₈ alkenyl group, a C₃ - C₁₈ aryl group, a C₃ - C₁₈ heteroalkyl group, a C₃ - C₁₈ heteroaryl group, an aralkyl group, an arylalkyl group, an aryl group, an heteroarylalkyl group, an alkoxy group, an acyl group, like a carbonylalkyl group or a carbonylaryl group, all of said groups may be substituted with at least one substituent independently selected from the group of C₁-C₆ alkyl, C₂-C₆ alkenyl, halogen, CN, NO₂, C₁-C₆ alkoxy, hydroxyl, carbamoyl, C₁ to C₈ alkylcarbamoyl, amido, C₁-C₆ alkoxycarbonyl, C₁-C₆ alkylcarbamoyloxy, C₁-C₆ alkoxycarbonylamino, amino, C₁-C₆ alkanoyloxy group, C₁-C₆ alkylthio and sulphur-containing analogs of oxygen containing substituents; Y is selected from an amide group, like an acetamide group, carbonyl group, carboxyl group, carbamoyl, alkoxy, amino, whereby Y and/or Z may be absent; or salts thereof, for use in the prophylaxis or treatment of hypertension and diseases, disorders or conditions due to or involving hypertension or high blood pressure.

The present inventors recognized that the triazolylpiperidine compounds of general formula III or IV are potent inhibitors of aspartic proteases, in particular, of the aspartic protease renin. Hence, the triazolylpiperidine compounds according to the present invention are useful in the prophylaxis or treatment of hypertension and diseases, disorders or conditions due to or involving hypertension or high blood pressure, like essential hypertension.

In another embodiment, the present invention relates to pharmaceutical compositions containing the triazolylpiperidine compounds of general formula III or IV together with suitable pharmaceutically acceptable excipients, diluents carrier.

### Brief description of the drawings

In figure 1 the results for inhibition of renin as an example for aspartic protease is shown. Identified is the remaining protease activity of human renin after incubation with the compounds according to the present invention at different concentrations.

Figure 2 shows preferred embodiments of the compounds according to the present invention. The reference numbers identity the compounds which synthesis is described in the examples.

### Detailed description of the present invention

In a first aspect, the present invention relates to a triazolylpiperidine compound of general formula III or IV 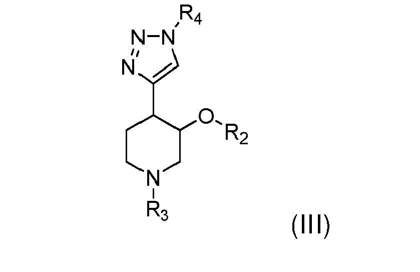 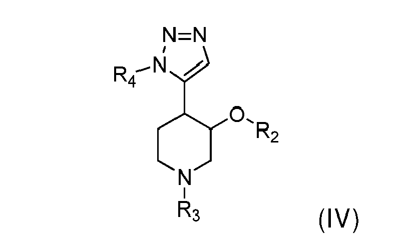 wherein R2 and R3 are independently from each other hydrogen or a C1 - C18 alkyl group, a C2 - C18 alkenyl group, a C3 - C18 aryl group, a C3 - C18 heteroalkyl group, a C3 - C18 heteroaryl group, an aralkyl group, an arylalkyl group, an aryl group, an heteroarylalkyl group, an alkoxy group, an acyl group, like a carbonylalkyl group or a carbonylaryl group, all of said groups may be substituted with at least one substituent independently selected from the group of C1-C6 alkyl, C2-C6 alkenyl, halogen, CN, NO2, C1-C6 alkoxy, hydroxyl, carbamoyl, C1 to C8 alkylcarbamoyl, amido, C1-C6 alkoxycarbonyl, C1-C6 alkylcarbamoyloxy, C1-C6 alkoxycarbonylamino, amino, C1-C6 alkanoyloxy group, C1-C6 alkylthio and sulphur-containing analogs of oxygen containing substituents;
R4 is selected from hydrogen or a C1 -C18 alkyl group, a C2 - C18 alkenyl group, a C3 - C18 aryl group, a C3 - C18 heteroalkyl group, a C3 - C18 heteroaryl group, an aralkyl group, an arylalkyl group, an aryl group, an heteroarylalkyl group, an alkoxy group, an acyl group, like a carbonylalkyl group or a carbonylaryl group, all of said groups may be substituted with at least one substituent independently selected from the group of C1-C6 alkyl, C2-C6 alkenyl, halogen, CN, NO2, C1-C6 alkoxy, hydroxyl, carbamoyl, C1 to C8 alkylcarbamoyl, amido, *N*-substituted amido, C1-C6 alkoxycarbonyl, C1-C6 alkylcarbamoyloxy, C1-C6 alkoxycarbonylamino, amino, C1-C6 alkanoyloxy group, C1-C6 alkylthio and sulphur-containing analogs of oxygen containing substituents, or a substituent of formula V

-X-Y-Z (V)

wherein X and Z are independently selected from a C₁ - C₁₈ alkyl group, a C₂ - C₁₈ alkenyl group, a C₃ - C₁₈ aryl group, a C₃ - C₁₈ heteroalkyl group, a C₃ - C₁₈ heteroaryl group, an aralkyl group, an arylalkyl group, an aryl group, an heteroarylalkyl group, an alkoxy group, an acyl group, like a carbonylalkyl group or a carbonylaryl group, all of said groups may be substituted with at least one substituent independently selected from the group of C₁-C₆ alkyl, C₂-C₆ alkenyl, halogen, CN, NO₂, C₁-C₆ alkoxy, hydroxyl, carbamoyl, C₁ to C₈ alkylcarbamoyl, amido, C₁-C₆ alkoxycarbonyl, C₁-C₆ alkylcarbamoyloxy, C₁-C₆ alkoxycarbonylamino, amino, C₁-C₆ alkanoyloxy group, C₁-C₆ alkylthio and sulphur-containing analogs of oxygen containing substituents; Y is selected from an amide group, like an acetamide group, carbonyl group, carboxyl group, carbamoyl group, alkoxy, in particular, C1-C6 alkoxy, amino, whereby Y and/or Z may be absent; or salts thereof, for use in the prophylaxis or treatment of hypertension and diseases, disorders or conditions due to or involving hypertension or high blood pressure.

As used herein, the term "hypertension" refers generally to describe high blood pressure. The term includes essential or primary hypertension as well as secondary hypertension. Secondary hypertension may be due to chronic kidney disease, disorders of the adrenal gland, pregnancy, medications such as birth control pills, diet pills, some cold medications and migraine medications, narrowed artery that supplies blood to the kidney or hyperparathyroidism. The term "hypertension" includes the prophylaxis or treatment of diseases, disorders or conditions which may occur due to high blood pressure including bleeding from the aorta, chronic kidney disease, heart attack and heart failure, poor blood supply to the legs, stroke, and problems with the vision.

In a preferred embodiment, the compounds according to the present invention are useful for treatment of hypertension, in particular, essential hypertension.

The term "C1 to C18" as used herein include compounds having C1, C2, C3, C4, C5, C6, C7, C8, C9, C10, C11, C12, C13, C14, C15, C16, C17, or C18 carbon atoms. The term "C1 to C6" include C1, C2, C3, C4, C5, C6 carbon atoms. The term "C2 to C6" include, C2, C3, C4, C5, C6 carbon atoms. The groups may be present in linear, branched or cyclic form.

"Alkyl" refers to a saturated aliphatic hydrocarbon including straight chain and branched chain groups. "Alkyl" may be exemplified by groups such as methyl, ethyl, n-propyl, isopropyl, n-butyl and the like. Alkyl groups may be substituted or unsubstituted. Substituents may also be themselves substituted. When substituted, the substituent group is preferably, but not limited to, C1-C4 alkyl, aryl, amino, cyano, halogen, alkoxy or hydroxyl.

"Alkenyl" refers to an unsaturated aliphatic hydrocarbon moiety including straight chain and branched chain groups. Alkenyl moieties contain at least one alkene. "Alkenyl" may be exemplified by groups such as ethenyl, n-propenyl, isopropenyl, n-butenyl and the like. Alkenyl groups may be substituted or unsubstituted. Substituents may also be themselves substituted. When substituted, the substituent group is preferably, but not limited to alkyl, halogen or alkoxy. Substituents may also be themselves substituted. Substituents may be located on the alkene itself and also on the adjacent member atoms of the alkenyl moiety.

"Alkynyl" refers to an unsaturated aliphatic hydrocarbon moiety including straight chain and branched chain groups. Alkynyl moieties contain at least one alkyne. "Alkynyl" may be exemplified by groups such as ethynyl, propynyl, n-butynyl and the like. Alkynyl groups may be substituted or unsubstituted. When substituted, the substituent group is preferably alkyl, amino, cyano, halogen, alkoxyl or hydroxyl. Substituents may also be themselves substituted. Substituents are not on the alkyne itself but on the adjacent member atoms of the alkynyl moiety.

"Acyl" or "carbonyl" refers to the group -C(O)R wherein R is H, alkyl, alkenyl, alkynyl, aryl, heteroaryl, carbocyclic, heterocarbocyclic, C1-C4 alkyl aryl or C1-C4 alkyl heteroaryl.

"Alkoxy" refers to the group -O-R wherein R is acyl, alkyl alkenyl, alkyl alkynyl, aryl, carbocyclic, heterocarbocyclic, heteroaryl, C1-C4 alkyl aryl or C1-C4 alkyl heteroaryl.

"Aralkyl" refers to a radical in which an aryl group is substituted for a hydrogen atom of an alkyl group; e.g., C6H5CH2-.

"Amino" refers to the group -NR'R" wherein R' and R" are each, independently, hydrogen, alkyl, aryl, heteroaryl, C1-C4 alkyl aryl or C1-C4 alkyl heteroaryl. The R' and R" groups may themselves be linked to form a ring.

"Aryl" refers to an aromatic carbocyclic group. "Aryl" may be exemplified by phenyl or benzyl or naphthyl. The aryl group may be substituted or unsubstituted. Substituents may also be themselves substituted. When substituted, the substituent group is preferably, but not limited to, alkyl, alkoxy, heteroaryl, acyl, carboxyl, amido, carbamoyl, carbonylamino, nitro, amino, cyano, halogen or hydroxyl. The substituents may be positioned at various locations on an aryl group. For example, substituents on a phenyl group may be located at an ortho-position, a meta-position, the para-position, or combinations thereof.

"Carboxyl" refers to the group -C(=O)OR, where R is a C1-C4 alkyl, aryl or heteroaryl. The alkyl or aryl group may be substituted or unsubstituted. Substituents may also be themselves substituted. When substituted, the substituent group is preferably, but not limited to, alkyl, alkoxy, heteroaryl, acyl, carboxyl, carbonylamino, nitro, amido, carbamoyl, amino, cyano, halogen or hydroxyl. The substituents may be positioned at various locations on an aryl group.

"Carbonyl" refers to the group -C(O)R wherein each R is, independently, hydrogen, alkyl, aryl, cycloalkyl, heterocycloalkyl, heteroaryl, C1-C4 alkyl aryl or C1-C4 alkyl heteroaryl.

"Heteroaryl" or "heteroaromatic" refers to a monocyclic or bicyclic aromatic carbocyclic radical having one or more heteroatoms in the carbocyclic ring. Heteroaryl may be substituted or unsubstituted. When substituted, the substituents may themselves be substituted. Preferred, but non-limiting substituents, are aryl, C1-C4 alkyl aryl, amino, halogen, hydroxyl, cyano, nitro, carboxyl, carbonylamino or C1-C4 alkyl. Preferred heteroaromatic groups include tetrazoyl, thiazolyl, thienyl, thiophenyl, thiazolyl, purinyl, pyrimidyl. pyridyl, and furanyl. More preferred heteroaromatic groups include benzothiofuranyl, thiophenyl, thienyl, furanyl, tetrazoyl, triazolyl, and pyridyl.

"Heteroatom" means an atom other than carbon in the ring of a heterocyclic group or a heteroaromatic group or the chain of a heterogeneous group. Preferably, heteroatoms are selected from the group consisting of nitrogen, sulfur, phosphor and oxygen atoms. Groups containing more than one heteroatom may contain different heteroatoms.

"Heterocarbocyclic group" or "heterocycloalkyl" or "heterocyclic" means a monovalent saturated or unsaturated hydrocarbon ring containing at least one heteroatom. Heterocarbocyclic groups are monocyclic, or are fused, spiro, or bridged bicyclic ring systems. Monocyclic heterocarbocyclic groups contain 3 to 10 carbon atoms, preferably 4 to 7 carbon atoms, and more preferably 5 to 6 carbon atoms in the ring. Bicyclic heterocarbocyclic groups contain 8 to 12 carbon atoms, preferably 9 to 10 carbon atoms in the ring. Heterocarocyclic groups may be substituted or unsubstituted. Suitable substituents include, but are not limited to, lower alkyl, hydroxyl, nitrile, halogen and amino. Substituents may also be themselves substituted. Preferred heterocarbocyclic groups include epoxy, tetrahydrofuranyl, azacyclopentyl, azacyclohexyl, piperidyl, and homopiperidyl. More preferred heterocarbocyclic groups include piperidyl, and homopiperidyl. The most preferred heterocarbocyclic group is piperidyl. Heterocarbocyclic groups are preferably not aromatic.

"Thioalkyl" refers to the group -S-alkyl.

The compound of general formula III represents a 1,4-disubstituted 1,2,3-triazolylpiperidine derivative while the compound of general formula III represents a 1,5-disubstituted 1,2,3-triazolylpiperidine derivative.

That is, the present inventors recognized that these triazolylpiperidine compounds are potent inhibitors of aspartic proteases, particularly of the enzyme renin.

In a preferred embodiment of the optionally substituted triazolylpiperidine compound according to the present invention the substituents at position 3 and position 4 of the piperidine ring are in trans position.

In a preferred embodiment of the present invention, the substituent R3 is hydrogen or a C1 to C6 alkyl group, more preferably, a hydrogen.

Moreover, it is preferred that the substituent R2 is selected from hydrogen, C1-C8 alkyl, an arylalkyl group, or an arylketo group. In particular, it is preferred that substituent R2 is an acetonaphthone group or a naphtyl group which may optionally be substituted as defined above, e.g. with a hydroxyl group, or a C1-C8 alkoxy group, in particular a methoxy group.

In another preferred embodiment, the compound is a compound wherein R4 is selected from a substituent of formula V

-X-Y-Z (V)

wherein X and Z are independently selected from a C₁ - C₁₈ alkyl group, a C₂ - C₁₈ alkenyl group, a C₃ - C₁₈ aryl group, a C₃ - C₁₈ heteroalkyl group, a C₃ - C₁₈ heteroaryl group, an aralkyl group, an arylalkyl group, an aryl group, an heteroarylalkyl group, an alkoxy group, an acyl group, like a carbonylalkyl group or a carbonylaryl group, all of said groups may be substituted with at least one substituent independently selected from the group of C₁-C₆ alkyl, C₂-C₆ alkenyl, halogen, CN, NO₂, C₁-C₆ alkoxy, hydroxyl, carbamoyl, C₁ to C₈ alkylcarbamoyl, amido, C₁-C₆ alkoxycarbonyl, C₁-C₆ alkylcarbamoyloxy, C₁-C₆ alkoxycarbonylamino, amino, C₁-C₆ alkanoyloxy group, C₁-C₆ alkylthio and sulphur-containing analogs of oxygen containing substituents; Y is selected from an amide group, like an acetamide group, carbonyl group, carboxyl group, carbamoyl, alkoxy, amino, whereby Y and/or Z may be absent.

It is particular preferred that the substituent R4 is selected from a phenyl group, a benzyl group, a phenethyl group whereby said groups may be substituted with an alkoxy group, in particular a methoxy group at position 4 of the aromatic ring, an acetamido group, an *N*-(naphthalene-2-ylmethyl)acetamido group, in particular an *N*-(naphthalene-2-ylmethyl)acetamido group at position 4 of the aromatic ring.

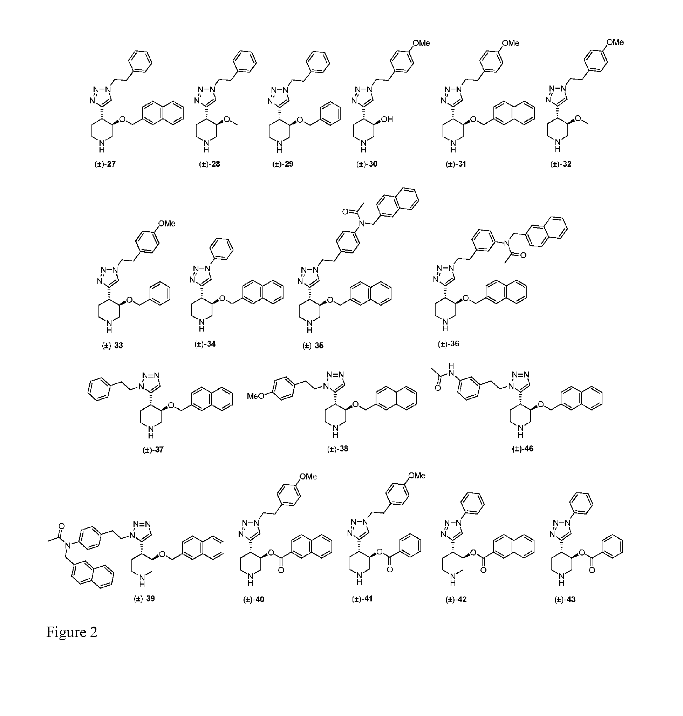

Particular preferred embodiments are shown in figure 2, namely, compounds:
(±)-*tran*s-3-(Naphthalene-2-ylmethoxy)-4-(1-phenethyl-1*H*-1,2,3-triazol-4-yl)piperidine;
(±)-*trans*-3-Methoxy-4-(1-phenethyl-1*H*-1,2,3-triazol-4-yl)piperidine;
(±)-*trans*-3-(Benzyloxy)-4-(1-phenethyl-1*H*-1,2,3-triazol-4-yl)piperidine;
(±)-*trans*-3-Hydroxy-4-(1-(4-methoxy-phenethyl)-1*H*-1,2,3-triazol-4-yl)piperidine;
(±)-*trans*-4-(1-(4-Methoxyphenethyl)-1*H*-1 ,2,3-triazol-4-yl)-3-(naphthalen-2-ylmethoxy)piperidine;
(±)-*trans*-3-Methoxy-4-(1-(4-methoxy-phenethyl)-1*H*-1,2,3-triazol-4-yl)piperidine;
(±)-*trans*-3-Benzyloxy-4-(1-(4-methoxy-phenethyl)-1*H*-1,2,3-triazol-4-yl)piperidine;
(±)-*trans*-3-(Naphthalen-2-ylmethoxy)-4-(1-phenyl-1*H*-1,2,3-triazol-4-yl)piperidine;
(±) *trans*-*N*-(4-(2-(4-(3-(Naphthalen-2-ylmethoxy)piperidin-4-yl)-1*H*-1,2,3-triazol-1-yl) ethyl)phenyl) )-*N*-(naphthalene-2-ylmethyl)acetamide;
(±) t*rans*-*N*-(3-(2-(4-(3-(Naphthalen-2-ylmethoxy)piperidin-4-yl)-1*H*-1,2,3-triazol-1-yl) ethyl)phenyl)-*N*-(naphthalene-2-ylmethyl)acetamide;
(±)-*trans*-3-(Naphthalen-2-ylmethoxy)-4-(1-phenethyl-1*H*-1,2,3-triazol-5-yl)piperidine;
(±)-*trans*-4-(1-(4-Methoxyphenethyl)-1*H*-1,2,3-triazol-5-yl)-3-(naphthalen-2-ylmethoxy)piperidine;
(±) *trans*-*N*-(4-(2-(5-(-3-(Naphthalen-2-ylmethoxy)piperidin-4-yl)-1*H*-1,2,3-triazol-1-yl)ethyl)phenyl)-*N*-(naphthalen-2-ylmethyl)acetamide;
(±)-*trans*-4-(1-(4-Methoxyphenethyl)-1*H*-1,2,3-triazol-4-yl)piperidin-3-yl 2-naphthoate;
(±)-*trans*-4-(1-(4-Methoxyphenethyl)-1*H*-1,2,3-triazol-4-yl)piperidin-3-yl 2-benzoate;
(±)-*trans*-4-(1-phenyl-1*H*-1,2,3-triazol-4-yl)piperidin-3-yl 2-naphthoate;
(±)-*trans*-4-(1-phenyl-1*H*-1,2,3-triazol-4-yl)piperidin-3-yl 2-benzoate;

Further, it is preferred that the substituent R4 is an arylether group, in particular, a phenethyl group optionally substituted with a methoxy group. Alternatively, R4 is a phenyl group optionally substituted with a methoxy group.

In another preferred embodiment, the compound of general formula III or IV is a compound wherein the substituent R2 is selected from the group of a hydrogen, a methyl group, a benzyl group, a naphtyl group, a carboxybenzyl group, a carboxynaphtyl group, a benzylmethyl group or a naphtalen-methyl group.

Moreover, in another preferred embodiment of the pharmaceutical composition comprising the optionally substituted triazolylpiperidine compound the compound is an inhibitor of aspartic proteases, preferably a renin inhibitor.

Preferably, the pharmaceutical composition comprising the optionally substituted triazolylpiperidine compound is intended for the treatment of humans and/or animals.

In another particular embodiment, the pharmaceutical composition comprises the optionally substituted triazolylpiperidine compound as racemate. In another preferred embodiment, the compounds are present as single enantiomers. Particularly preferred, the (3R, 4R) enantiomers are used. The skilled person is well aware of suitable methods for preparing and isolating single enantiomers from racemic mixtures. Unless otherwise indicated, the compounds relates to the racemate as well as to the single enantiomers thereof.

Another embodiment of the present inventions relates to a pharmaceutical composition containing the compound according to the present invention for use in the prophylaxis and treatment of hypertension or diseases, disorders or conditions due to or involving hypertension or high blood pressure. The skilled person is well aware of suitable diluents, excipients, or carriers.

The pharmaceutical composition may be administered with a physiologically acceptable carrier to a patient, as described herein. In a specific embodiment, the term "pharmaceutically acceptable" means approved by a regulatory agency or other generally recognized pharmacopoeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatine, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations, patches and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium, carbonate, etc. Examples of suitable pharmaceutical carriers are described in "Remington's Pharmaceutical Sciences" by E.W. Martin (18th ed., Mack Publishing Co., Easton, PA (1990)). Such compositions will contain a therapeutically effective amount of the aforementioned compounds according ot the present invention, preferably in purified form, together with a suitable amount of carrier so as to provide the form for proper administration to the patient. The formulation should suit the mode of administration.

Typically, pharmaceutically or therapeutically acceptable carrier is a carrier medium which does not interfere with the effectiveness of the biological activity of the active ingredients and which is not toxic to the host or patient.

In another preferred embodiment, the composition is formulated in accordance with routine procedures as a pharmaceutical composition adapted for intravenous or oral administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in a unit dosage form, for example, as a dry lyophilised powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The pharmaceutical composition for use in connection with the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

"Therapeutically- or pharmaceutically-effective amount" as applied to the compositions of the instant invention refers to the amount of composition sufficient to induce a desired biological result. That result can be alleviation of the signs, symptoms, or causes of a disease, or any other desired alteration of a biological system. In the present invention, the result will typically involve a decrease in the immunological and/or inflammatory responses to infection or tissue injury and/or decreased tumor growth and/or tumor volume decrease, and/or tumor necrosis, and/or tumor apoptosis.

In vitro assays may optionally be employed to help identifying optimal dosage ranges. The precise dose to be employed in the formulation will also depend on the route of administration, and the seriousness of the disease or disorder, and should be decided according to the judgement of the practitioner and each patient's circumstances. Effective doses may be extrapolated from dose-response curves derived from in vitro or animal model test systems. Preferably, the pharmaceutical composition is administered directly or in combination with an adjuvant. The exact dose will depend on the purpose of the treatment, and will be ascertainable by one skilled in the art using known techniques. As is known in the art and described above, adjustments for systemic versus localized delivery, age, body weight, general health, sex, diet, time of administration, drug interaction and the severity of the condition may be necessary, and will be ascertainable with routine experimentation by those skilled in the art.

In the context of the present invention the term "subject" means an individual in need of a therapy that can be alleviated or cured by administering the compounds according to the present invention to the individual. Preferably, the subject is a vertebrate, even more preferred a mammal, particularly preferred a human.

The term "administered" means administration of a therapeutically effective dose of the aforementioned pharmaceutical composition comprising the compounds according to the present invention.

The methods are applicable to both human therapy and veterinary applications. The compounds described herein having the desired therapeutic activity may be administered in a physiologically acceptable carrier to a patient, as described herein. Depending upon the manner of introduction, the compounds may be formulated in a variety of ways as discussed below. The concentration of therapeutically active compound in the formulation may vary from about 0.1-100 wt%. The agents may be administered alone or in combination with other treatments.

The administration of the pharmaceutical composition can be done in a variety of ways as discussed above, including, but not limited to, orally, subcutaneously, intravenously, intra-arterial, intranodal, intramedullary, intrathecal, intraventricular, intranasally, intrabronchial, transdermally, intrarectally, intraperitoneally, intramuscularly, intrapulmonary, vaginally, rectally, or intraocularly. In some instances, for example, in the treatment of wounds and inflammation, the pharmaceutically effective agent may be directly applied as a solution dry spray.

The attending physician and clinical factors will determine the dosage regimen. A typical dose can be, for example, in the range of 0.001 to 1000 µg; however, doses below or above this exemplary range are envisioned, especially considering the aforementioned factors.

That is, the present invention provides antihypertensive drugs according to general formula III or IV as defined herein. It is preferred that the antihypertensive drug is in a form of a pharmaceutical composition, in particular for oral administration. In addition, unless otherwise defined herein, the compounds of general formula III or IV includes salts and solvays thereof.

Moreover, it is noted that the term "comprising" or "containing" includes the specific embodiment of "consisting of".

### Examples

The following examples have been included to illustrate modes of the present disclosed subject matter. In light of the present disclosure and the general level of the skilled in the art, those of skilled will appreciate that the following examples are intended to be exemplary only and that numerous changes, modifications and alterations can be employed without departing from the scope of the present disclosed subject matter.

A suitable method for the synthesis of compounds of general formula III or IV according to the present invention is depicted in the scheme below:

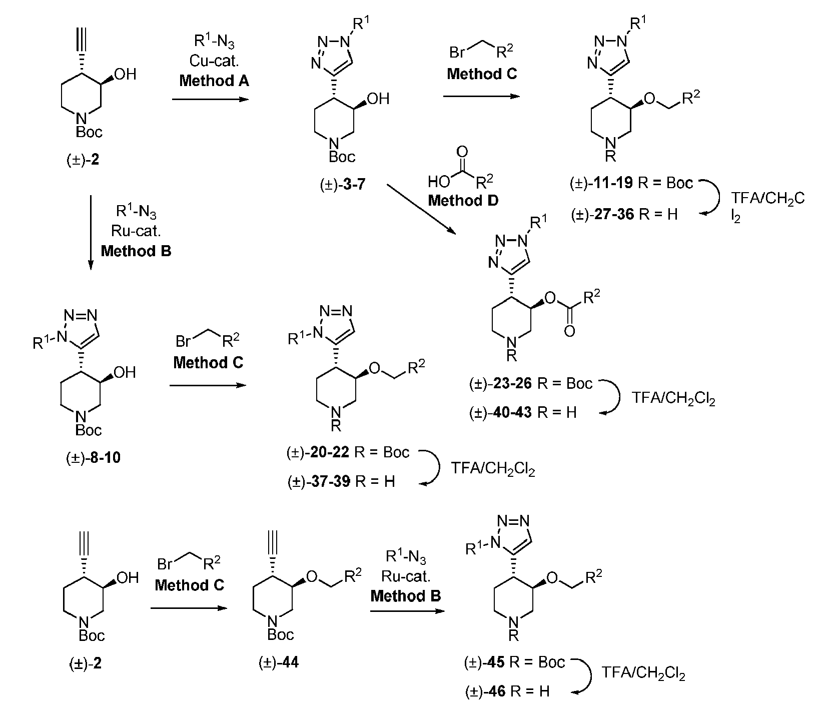

A detailed description is provided e.g. in EP 10194913.9 which is enclosed herewith by reference.

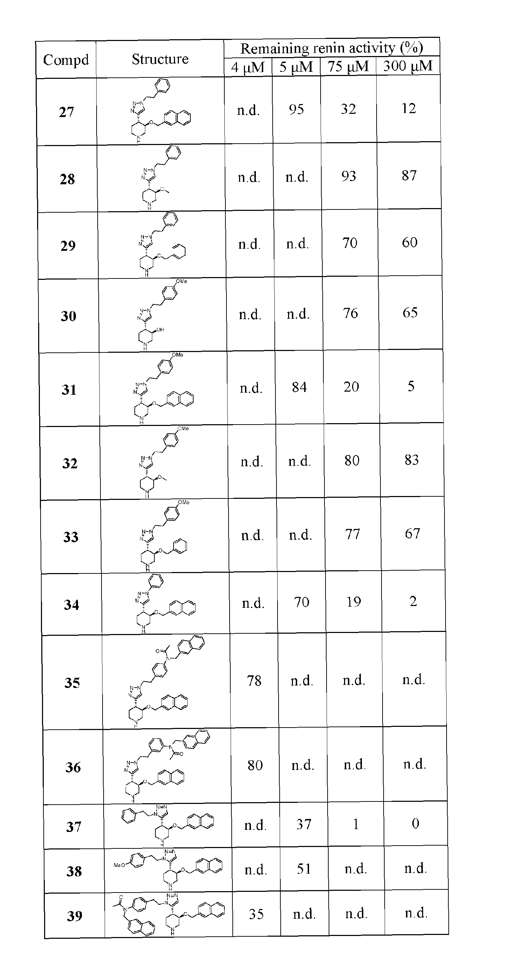

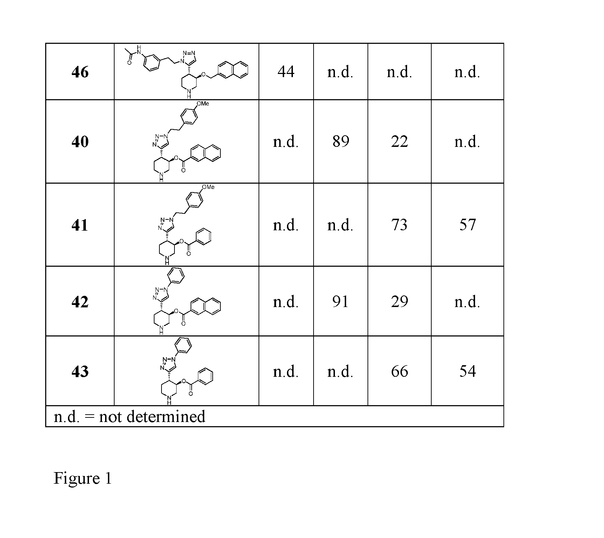

In the following, the synthesis of the compounds as shown in figure 1 and in the scheme above and tested herein is described:

### Experimental

All starting materials, reagents and solvents were obtained from Sigma-Aldrich and used without purification unless otherwise noted. Anhydrous solvents were obtained from the Department of Chemistry University of Bergen anhydrous solvent delivery system (mBraun SPS-800 system). Flash column chromatography was performed using silica gel for flash chromatography from J.T. Baker ("Baker Analyzed", 0.063 - 0.200 mm) supplied by Chiron AS (Norway). TLC analysis was done using Macherey-Nagel aluminum plates coated with silica gel 60 with fluorescent indicator UV₂₅₄ supplied by Chiron AS (Norway) with visualization using ultraviolet light or a solution of 2% ninhydrin in ethanol containing 10 drops of conc. sulfuric acid pr. 100 mL of the solution. Purification by reversed-phase high performance liquid chromatography (RP-HPLC) was performed using a C₁₈-column (Ascentis^{®} C18, 5 µm, 21.2x250 mm, Supelco Corp., Bellefonte, PA, USA) with a mixture of water and acetonitrile (both containing 0.1 % TFA) as mobile phase and UV-detection at 220 nm. Analytical RP-HPLC was performed using a C₁₈-column (Ascentis^{®} C18, 5 µm, 4.6x250 mm, Supelco Corp., Bellefonte, PA, USA).

NMR spectra were recorded using a Bruker Spectrospin AC. The chemical shifts are reported in parts per million relative to TMS and the couplings constants are given in Hz. ¹³C peaks marked with * were identified from cross peaks in HSQC spectra. Accurate mass determinations were performed using a Thermo Scientific LTQ Orbitrap MS.

### Synthesis of bromides

### N-(4-(2-Bromoethyl)phenyl)acetamide

4-Nitrophenylethyl bromide (0.1 g, 0.43 mmol) was dissolved in a mixture of acetic acid (1.6 mL) and methanol (0.75 mL) and heated to 65 ºC. Fe powder (87 mg) was added slowly to this solution. The reaction mixture was stirred at 65 ºC for 20 hours after which the solvent was removed under reduced pressure. The resulting residue was coevaporated with toluene. CH₂Cl₂ (10 mL) was added to the residue and the obtained solution was washed with concentrated NH₃ solution (5 mL) and brine (5 mL). The organic phase was dried over Na₂SO₄ and the solvent removed under reduced pressure. The resulting product was dissolved in dry CH₂Cl₂ (0.4 mL). Et₃N (0.13 mL, 0.95 mmol) and acetyl chloride (1.6 mL, 0.95 mmol) were added and the mixture was stirred under reflux for 2.5 hours. The reaction mixture was quenched with 0.1 N NaOH (1 mL) and the obtained aqueous mixture was extracted with CH₂Cl₂ (5 mL). The organic phase was washed with brine, dried (MgSO₄) and the solvent removed under reduced pressure. Purification by flash column chromatography (hexane-EtOAc, 2:1) gave the title compound as a yellow solid (17 mg, 17%); mp 86.5-87.3;
R_{f} = 0.22 (hexane/EtOAc, 2:1); ¹H NMR (400 MHz, CDCl₃) δ 7.45 (d, *J* = 8.3, 1 H), 7.26 (s, 1H), 7.16 (d, *J* = 8.2, 1H), 3.54 (t, *J* = 7.6, 1H), 3.12 (t, *J* = 7.5, 1 H), 2.17 (s, 1H); ¹³C NMR (101 MHz, CDCl₃) δ 168.86, 137.05, 135.11, 129.48, 120.50, 39.05, 33.38, 24.82; HRMS (ESI): *m*/*z* [M + H]⁺ calcd for C₁₀H₁₃BrNO: 242.0184; found:242.0184 and 244.0161.

### N-(3-(2-Bromoethyl)phenyl)acetamide

The title compound was prepared from 3-nitrophenylethyl bromide (1.1 g, 4.78 mmol) as described for *N*-(4-(2-bromoethyl)phenyl)acetamide. Purification by flash column chromatography (hexane-EtOAc, 2:1) gave the title compound as a yellow solid (0.347 g, 30%); mp. 136.0-137.3; R_{f} = 0.2 (hexane/EtOAc, 2:1); ¹H NMR (400 MHz, CDCl₃) δ 7.45 (s, 1H), 7.38 - 7.23 (m, 3 H), 6.96 (d, *J* = 7.4, 1H), 3.56 (t, *J* = 7.5, 2 H), 3.14 (t, *J* = 7.5, 2 H), 2.18 (s, 1H); ¹³C NMR (101 MHz, CDCl₃) δ 168.84, 140.16, 138.50, 129.48, 124.91, 120.39, 118.69, 39.57, 33.11, 24.93; HRMS (ESI): *m*/*z* [M + Na]⁺ calcd for C₁₀H₁₂BrNONa: 263.9996; found: 264.0000.

### Synthesis of azides

A bromide (1 mmol) was dissolved in DMSO (1 mL) and a mixture of NaN₃ in DMSO (0.5M, 2.2 mL, 1.1 mmol)) was added. The resulting mixture was stirred at rt over night before the reaction was quenched with H₂O (25 mL). The aqueous mixture was extracted three times with Et₂O (3 x 15 mL). The organic layers were combined, washed twice with H₂O (2 x 25 mL) and once with a saturated aqueous solution of NaCl (25 mL). The organic layer was dried over MgSO₄, filtered and the solvent was removed under reduced pressure. The crude product was purified by flash column chromatography.
2-(azidoethyl)benzene was prepared as previously described in the literature¹.
1-(2-azidoethyl)-4-methoxybenzene was prepared following a literature procedure¹.

### Azidobenzene

A suspension of aniline (0.5 mL, 5.49 mmol) in acetic acid and conc. sulphuric acid (ratio 2.25:1, 6 mL) was cooled to 0 ºC in an ice bath. A mixture of NaNO₂ (2 M in H₂O, 2.9 mL) was added drop wise to the suspension and the resulting mixture was stirred at 0 ºC for 1 h. To the obtained solution, urea (2M in H₂O, 2.9 mL) and NaN₃ (2.5 M in H₂O, 2.4 mL) were added and the mixture was stirred for another 3 h at 0 ºC. The reaction was quenched with ice cold H₂O (50 mL) and basified with 40% NaOH (aq). This aqueous mixture was extracted with hexane (3 x 50 mL), dried over MgSO₄, filtered and the solvent was removed under reduced pressure. The crude product was purified by flash column chromatography (hexane) to give the title compound as a yellow liquid (0.49 g, 75%); R_{f} = 0.53 (hexane). Analytical data is in accordance with those reported in the literature².

### N-(4-(2-Azidoethyl)phenyl)acetamide

The title compound was prepared from 4-nitrophenylethyl bromide (0.153 g, 0.63 mmol) according to the general method for the synthesis of azides to give a yellow solid (0.11 g, 86%); mp. 89.2-90.8; R_{f} = 0.20 (hexane/EtOAc, 2:1); ¹H NMR (400 MHz, CDCl₃) δ 8.79 (s, 1 H), 7.52 (s, 1 H), 7.39 (d, *J* = 7.8, 1 H), 7.22 (t, *J* = 7.8, 1 H), 6.93 (d, *J* = 7.5, 1 H), 3.47 (t, *J* = 7.2, 2 H), 2.84 (t, *J* = 7.2, 2 H), 2.14 (s, 3 H); ¹³C NMR (101 MHz, CDCl₃) δ 168.55, 136.73, 134.11, 129.41, 120.33, 52.59, 34.90, 24.68; *m*/*z* [M + H]⁺ calcd for C₁₀H₁₃N₄O:205.1089; found: 205.1104.

### N-(3-(2-Azidoethyl)phenyl)acetamide

The title compound was prepared from bromide *N*-(3-(2-bromoethyl)phenyl)acetamide (0.107 g, 0.44 mmol) as described in the general procedure for the preparation of azides to give a colourless oil (71 mg, 80%); R_{f} = 0.33 (hexane/EtOAc, 1:1); ¹H NMR (400 MHz, CDCl₃) δ 8.79 (s, 1 H), 7.52 (s, 1 H), 7.39 (d, *J* = 7.8, 1 H), 7.22 (t, *J* = 7.8, 1 H), 6.93 (d, *J* = 7.5, 1 H), 3.47 (t, *J* = 7.2, 2 H), 2.84 (t, *J* = 7.2, 2 H), 2.14 (s, 3 H); ¹³C NMR (101 MHz, CDCl₃) δ 168.83, 139.36, 138.57, 129.51, 124.97, 120.47, 118.53, 52.59, 35.60, 24.91; HRMS (ESI): *m*/z [M + Na]⁺ calcd for C₁₀H₁₂N₄NaO: 227.0909; found: 227.0897

### General procedure for copper catalyzed cycloadditions - Method A.

To a solution of alkyne **2** (1.0 mmol) and an azide (1.0 mmol) in a mixture of H₂O and CH₂Cl₂ (1:1, 0.05 mmol/mL) were added CuSO₄˙5 H₂O (0.05 mmol) and sodium ascorbate (0.15 mmol). The reaction mixture was stirred at room temperature until TLC showed full conversion of the starting material after which the mixture was filtered and the precipitate was washed with H₂O. The filtrate was extracted with EtOAc (3 x 10 mL) and the organic layers were combined with the precipitate. These combined layers were washed with H₂O (10 mL) and a saturated aqueous solution of NaCl (10 mL), dried over MgSO₄, filtered, and the solvent was removed under reduced pressure. The crude product was purified by flash column chromatography.
(±)-*tert*-Butyl *trans*-3-hydroxy-4-(1-phenethyl-1*H*-1,2,3-triazol-4-yl)piperidine-1-carboxylate (**3**) was prepared as previously reported³.
(±)-*tert*-Butyl 3-hydroxy-4-(1-(4-methoxyphenethyl)-1*H*-1,2,3-triazol-4-yl)piperidine-1-carboxylate (**4**) was prepared as previously reported³.

### (±)-tert-Butyl trans-3-hydroxy-4-(1-phenyl-1H-1,2,3-triazol-4-yl)piperidine-1-carboxylate (5)

The title compound was prepared from alkyne **2** (0.308 g, 1.37 mmol) and azidobenzene (0.163 g, 1.37 mmol) using the general method A. The reaction mixture was stirred at room temperature for 3 hrs after which TLC analysis indicated full conversion of the starting material. The crude product was purified by flash column chromatography (hexane-EtOAc, 1:1) to give the title compound as a colourless solid (0.420 g, 89%); mp 130.4-132.3; R_{f} = 0.19 (hexane-EtOAc, 1:1); ¹H NMR (400 MHz, CDCl₃): δ 7.83 (s, 1 H), 7.71 (d, *J* = 7.95, 2 H), 7.51 (t, *J* = 7.47, 2 H), 7.48-7.41 (m, 1 H), 4.48-4.33 (m, 1 H), 4.31-4.07 (m, 1 H), 3.83-3.69 (m, 1 H), 2.94-2.66 (m, 3 H), 2.15-2.05 (m, 1 H), 1.85-1.69 (m, 1 H), 1.47 (s, 9 H); ¹³C NMR (101 MHz, CDCl₃): δ 155.05, 146*, 137.52, 130.11, 129.15, 120.94, 119.30, 80.28, 70.69, 49.98, 43.63, 41.76, 29.66, 28.80; HRMS (ESI): *m*/*z* [M + Na]⁺ calcd for C₁₈H₂₄N₄NaO₃: 367.1746; found: 367.1740.

### (±) tert-Butyl trans-4-(1-(4-acetamidophenethyl)-1H-1,2,3-triazol-5-yl)-3-hydroxypiperidine-1-carboxylate (6)

The title compound was prepared from alkyne 2 (43 mg, 0.19 mmol) and *N*-(4-(2-azidoethyl)phenyl)acetamide (43 mg, 0.21 mmol) following the general procedure for Ru(II)-catalyzed 1,3-dipolar cycloadditions. After 21 h at 80 ºC, TLC analysis indicated full conversion of the starting material. Purification by flash column chromatography (EtOAc) gave triazole **6** as a colourless solid (73 mg, 90%); mp 208.7 - 209.5; R_{f} = 0.24 (EtOAc); ¹H NMR (400 MHz, CDCl₃) δ 7.36 (d, *J* = 8.0, 2 H), 7.31 - 7.13 (m, 2 H), 6.98 (d, *J* = 8.1, 2 H), 4.57 (t, *J* = 7.4, 3 H), 4.30 - 4.18 (m, 1 H), 4.09 - 3.90 (m, 1 H), 3.53 - 3.35 (m, 1 H), 3.24 - 3.03 (m, 2 H), 2.74 - 2.42 (m, 3 H), 2.23 - 1.98 (m, 4 H), 1.46 (s, 9 H), 1.38 - 1.32 (m, 1 H); ¹³C NMR (101 MHz, CDCl₃) δ 170.21, 156.09, 147*, 135.62, 130.54, 131.12, 129.73, 120.56, 80.61, 71.47, 50*, 49.33, 44*, 40*, 36.46, 29*, 28.61, 24.48; HRMS (ESI): *m*/*z* [M + H]⁺ calcd for C₂₂H₃₂N₅O₄: 430.2454; found: 430.2459.

### (±) tert-Butyl trans-4-(1-(3-acetamidophenethyl)-1H-1,2,3-triazol-5-yl)-3-hydroxypiperidine-1-carboxylate (7)

The title compound was synthesized from alkyne **2** (0.12 g, 0.33 mmol) and *N*-(3-(2-azidoethyl)phenyl)acetamide (74 mg, 0.36 mmol) following the general procedure for Ru(II)-catalyzed 1,3-dipolar cycloadditions. The reaction mixture was stirred at 80 °C for 21 h after which TLC analysis indicated full conversion of the starting material. Purification by flash column chromatography (hexane-EtOAc, 1 :9) gave the title compound as a colourless oil (0.197 g, 82%); R_{f} = 0.26 (hexane-EtOAc, 1:9); ¹H NMR (400 MHz, CDCl₃) δ 7.64 (s, 1 H), 7.52 - 7.00 (m, 3 H), 6.80 (d, *J* = 7.4, 1 H), 4.61 (t, *J* = 6.7, 2 H), 4.36- 4.18 (m, 1 H), 4.20 - 3.95 (m, 1 H), 3.49 - 3.34 (m, 1 H), 3.30 - 3.05 (m, 2 H), 2.60 - 2.44 (m, 2 H), 2.26 - 2.20 (m, 1 H), 2.15 (s, 3 H), 1.55 - 1.34 (m, 10 H), 1.39 - 1.16 (m, 1 H). ¹³C NMR (400 MHz, CDCl₃) δ 169.02, 154.77, 150*, 138.87, 138*, 137*, 130*, 129.66, 125.21, 118.93, 80.54, 71.43, 50*, 49.52, 43*, 36.87, 30.46, 28.74, 24.88; HRMS (ESI): *m*/*z* [M + H]⁺ calcd for C₂₂H₃₂N₅O₄: 430.2454; found: 430.2457.

### General procedure for ruthenium catalyzed cycloadditions - Method B.

A solution of alkyne **2** (1 mmol) in dry THF (15 mL/mmol) was heated in an oil bath to 80 °C. To this mixture Cp*RuCI(PPh₃)₂ (0.01 mmol) and azide (1.1 mmol) were added. The reaction mixture was stirred at 80 °C until TLC analysis showed full conversion of the starting material. The solvent was removed using a rotary evaporator. The crude reaction mixture was purified by flash column chromatography.

(±)-*tert*-Butyl *trans*-3-hydroxy-4-(1-phenethyl-1*H*-1,2,3-triazol-5-yl)piperidine-1-carboxylate (**8**) was prepared as previously reported.³ (±)-*tert*-Butyl *trans*-3-hydroxy-4-(1-(4-methoxyphenethyl)-1*H*-1,2,3-triazol-5-yl)piperidine-1-carboxylate (**9**)

The title compound was prepared from alkyne **2** (0.114 g, 0.51 mmol) and 1-(2-azidoethyl)-4-methoxybenzene (98 mg, 0.56 mmol) using Method B. The reaction mixture was stirred for 20 hrs at 80 °C after which TLC analysis indicated full conversion of the starting material. Purification by flash column chromatography (hexane-EtOAc, 1:4) gave **9** as a colourless solid (0.173 g, 84%); 142.4-144.3; R_{f} = 0.3 (hexane-EtOAc, 1:4); ¹H NMR (400 MHz, CDCl₃) δ 7.28 (s, 1 H), 6.94 (d, *J* = 8.3, 2 H), 6.79 (d, *J* = 8.3, 2 H), 4.65 - 4.46 (m, 2 H), 4.31 - 4.19 (m, 1 H), 4.10 - 3.98 (m, 1 H), 3.75 (s, 3 H), 3.48 - 3.37 (m, 1 H), 3.14 (t, *J* = 7.6, 2 H), 2.65 (s, 1 H), 2.57 - 2.42 (m, 2 H), 2.24 - 2.13 (m, 1 H), 1.53 - 1.43 (m, 10 H), 1.39 - 1.28 (m, 1 H); ¹³C NMR (101 MHz, CDCl₃) δ 158.96, 154.72, 139.57, 130.45, 130.32, 129.97, 114.43, 80.53, 71.62, 55.66, 51*, 50.07, 44*, 40.52, 36.39, 30.25, 28.74; HRMS (ESI): *m*/*z* [M + Na]⁺ calcd for C₂₁H₃₀N₄O₄Na: 425.2165; found: 425.2159.

### (±) tert-Butyl trans-4-(1-(4-acetamidophenethyl)-1H-1,2,3-triazol-5-yl)-3-hydroxypiperidine-1-carboxylate (10)

The title compound was prepared from alkyne **2** (43 mg, 0.19 mmol) and *N*-(4-(2-azidoethyl)phenyl)acetamide (43 mg, 0.21 mmol) using Method B. After 21 h at 80 ºC, TLC analysis indicated full conversion of the starting material. Purification by flash column chromatography (EtOAc) gave triazole **4.22** as a colourless solid (73 mg, 90%); mp 208.7 - 209.5; R_{f} = 0.24 (EtOAc); ¹H NMR (400 MHz, CDCl₃) δ 7.36 (d, *J* = 8.0, 2 H), 7.31 - 7.13 (m, 2 H), 6.98 (d, *J* = 8.1, 2 H), 4.57 (t, *J* = 7.4, 3 H), 4.30 - 4.18 (m, 1 H), 4.09 - 3.90 (m, 1 H), 3.53 - 3.35 (m, 1 H), 3.24 - 3.03 (m, 2 H), 2.74 - 2.42 (m, 3 H), 2.23 - 1.98 (m, 4 H), 1.46 (s, 9 H), 1.38 - 1.32 (m, 1 H); ¹³C NMR (101 MHz, CDCl₃) δ 170.21, 156.09, 147*, 135.62, 130.54, 131.12, 129.73, 120.56, 80.61, 71.47, 50*, 49.33, 44*, 40*, 36.46, 29*, 28.61, 24.48; HRMS (ESI): *m*/*z* [M + H]⁺ calcd for C₂₂H₃₂NO₄: 430.2454; found: 430.2459.

### General procedure for the formation of ethers linkages - Method C

To a secondary alcohol (0.2 mmol) was added to a suspension of NaH (60 wt% in mineral oil, 0.4 mmol) in DMF (5 mL). After stirring for 30 min at room temperature, a mixture of TBAI (0.02 mmol) and an alkyl bromide (0.2 mmol) in DMF (1 mL) was added and the reaction mixture was stirred at room temperature until TLC analysis indicated full conversion of the alcohol. The reaction was quenched by addition of H₂O (10 mL) and the aqueous mixture was extracted with EtOAc (20 mL). The organic layer was washed two times each with 10% solution of HCl (2 x 10 mL), a saturated aqueous solution of NaHCO₃ (2 x 10 mL) and H₂O (2 x 10 mL), and dried over MgSO₄. After removal of the drying agent by filtration, the solvent was removed under vacuum. The crude product was purification by flash column chromatography.

### (±)-tert-Butyl trans-3-(naphthalene-2-ylmethoxy)-4-(1-phenethyl-1H-1,2,3-triazol-4-yl)piperidine-1-carboxylate (11)

The title compound was prepared from alcohol 3 (55 mg, 0.15 mmol) and 2-(bromomethyl)naphthalene (66 mg, 0.30 mmol) using Method C. The reaction mixture was stirred for 20 hours after which TLC analysis indicated full conversion of the starting material. Purification by flash column chromatography (hexane-EtOAc, 1:1) gave **11** as a colourless oil (39 mg, 70%); R_{f} = 0.18 (hexane-EtOAc, 1:1); ¹H NMR (400 MHz, CDCl₃): δ 7.83-7.74 (m, 3 H), 7.60 (s, 1 H), 7.48-7.40 (m, 2 H), 7.29-7.15 (m, 4 H), 7.05-6.97 (m, 2 H), 6.93 (s, 1 H), 4.78-4.63 (m, 1 H), 4.54-4.29 (m, 4 H), 4.06-3.97 (m, 1 H), 3.63-3.52 (m, 1 H), 3.09 (t, *J* = 7.12, 2 H), 2.96-2.75 (m, 3 H), 2.09-1.98 (m, 1 H), 1.89-1.76 (m, 1 H), 1.46 (s, 9 H); ¹³C NMR (101 MHz, CDCl₃): δ 155.04, 148.65, 137.55, 136.21, 133.69, 133.41, 129.09, 128.97, 128.35, 128.26, 128.02, 127.38, 126.86, 126.48, 126.27, 126.25, 121.95, 80.14, 77.61, 72.20, 51.66, 47*, 43*, 40.38, 37.04, 30.49, 28.80; HRMS (ESI): m/z [M + Na]⁺ calcd for C₃₁H₃₆N₄O₃Na: 535.2685; found: 535.2693.

### (±)-tert-Butyl trans-3-methoxy-4-(1-phenethyl-1H-1,2,3-triazol-4-yl)piperidine-1-carboxylate (12)

The title compound was prepared from alcohol 3 (94 mg, 0.25 mmol) and methyl iodide (31 µL, 0.50 mmol) using Method C. The reaction mixture was stirred for 22 hours after which TLC analysis indicated full conversion of the starting material. Purification by flash column chromatography (hexane-EtOAc, 4:1) gave **12** as a colourless oil (90 mg, 93%); R_{f} = 0.30 (hexane-EtOAc, 1:4); ¹H NMR (400 MHz, CDCl₃): δ 7.32-7.21 (m, 3 H), 7.12-7.05 (m, 3 H), 4.57 (t, *J* = 7.14, 2 H), 4.52-4.12 (m, 1 H), 4.05-3.96 (m, 1 H), 3.32-3.24 (m, 1 H), 3.25 (s, 3 H), 3.20 (t, *J* = 7.20, 2 H), 2.89-2.72 (m, 2 H), 2.71-2.50 (m, 1 H), 2.07-1.97 (m, 1 H), 1.88-1.74 (m, 1 H), 1.47 (s, 9 H); ¹³C NMR (101 MHz, CDCl₃): δ 155.00, 148.51, 137.54, 129.11, 129.05, 127.39, 121.97, 80.16, 79.89, 57.61, 51.83, 47*, 43*, 40.08, 37.13, 30.35, 28.78; HRMS (ESI): *m*/*z* [M + Na]⁺ calcd for C₂₁H₃₀N₄O₃Na: 409.2216; found: 409.2212.

### (±)-tert-Butyl trans-3-(benzyloxy)-4-(1-phenethyl-1H-1,2,3-triazol-4-yl)piperidine-1-carboxylate (13)

The title compound was prepared from alcohol **3** (60 mg, 0.18 mmol) benzyl bromide (43 µL, 0.36 mmol) using Method C. The reaction mixture was stirred for 20 hours after which TLC analysis indicated full conversion of the starting material. Purification by flash column chromatography (hexane-EtOAc, 2:1) gave **13** as a colourless oil (53 mg, 64%); R_{f} = 0.33 (hexane-EtOAc, 1:2); ¹H NMR (400 MHz, CDCl₃): δ 7.32-7.19 (m, 6 H), 7.14 (d, *J* = 6.86, 2 H), 7.08 (d, *J* = 6.68, 2 H), 7.00 (s, 1 H), 4.61-4.42 (m, 4 H), 4.31 (d, *J* = 11.08, 1 H), 4.02 (d, *J* = 13.24, 1 H), 3.58-3.49 (m, 1 H), 3.15 (t, *J* = 7.24, 2 H), 2.93-2.56 (m, 3 H), 2.08-1.97 (m, 1 H), 1.89.1,76 (m, 1 H), 1.47 (s, 9 H); ¹³C NMR (101 MHz, CDCl₃): δ 154.96, 148.52, 138.56, 137.49, 129.12, 129.00, 128.64, 128.12, 128.00, 127.40, 122.02, 80.14, 77.39, 72.04, 51.76, 48*, 43*, 40.22, 37.12, 30.42, 28.79; HRMS (ESI): *m*/*z* [M + Na]⁺ calcd for C₂₇H₃₄N₄O₃Na: 485.2529; found: 485.2526.

### (±)-tert-Butyl trans-4-(1-(4-methoxy-phenethyl)-1H-1 ,2,3-triazol-4-yl)-3-(naphthalene-2-ylmethoxy)piperidine-1-carboxylate (14)

The title compound was prepared from alcohol **4** (0.229 g, 0.57 mmol) and 2-(bromomethyl)naphthalene (0.252 g, 1.14 mmol) using Method C. The reaction mixture was stirred for 21 hours after which TLC analysis indicated full conversion of the starting material. Purification by flash column chromatography (hexane-EtOAc, 1:1) gave **14** as a colourless oil (0.245 g, 79%); R_{f} = 0.17 (hexane-EtOAc, 1:1); ¹H NMR (400 MHz, CDCl₃): δ 7.83-7.71 (m, 3 H), 7.61 (s, 1 H), 7.49-7.21 (m, 2 H), 7.27-7.21 (m, 1 H), 6.95 (s, 1 H), 6.91 (d, *J* = 8.48, 2 H), 6.74 (d, *J* = 8.48, 2 H), 4.77-4.62 (m, 1 H), 4.53-4.30 (m, 4 H), 4.07-3.97 (m, 1 H), 3.71 (s, 3 H), 3.64-3.52 (m, 1 H), 3.02 (t, *J* = 7.24, 2 H), 2.96-2.74 (m, 3 H), 2.09-1-96 (m, 1 H), 1.90-1.76 (m, 1 H), 1.44 (s, 9 H); ¹³C NMR (101 MHz, CDCl₃): δ 158.91, 154.94, 148.51, 136.01, 133.53, 133.27, 129.96, 129.39, 128.33, 128.22, 127.98, 126.85, 126.47, 126.27, 126.22, 121.99, 114.44, 80.14, 77*, 72.16, 55.53, 51.91, 48*, 44*, 40.32, 36.19, 30.51, 28.73; HRMS (ESI): *m*/*z* [M + Na]⁺ calcd for C₃₂H₃₈N₄O₄Na: 565.2791; found: 565.2772.

### (±)-tert-Butyl trans-3-methoxy-4-(1-(4-methoxyphenethyl)-1H-1,2,3-triazol-4-yl)piperidine-1-carboxylate (15)

The title compound was prepared from alcohol **4** (0.129 g, 0.32 mmol) and methyl iodide (40 µL, 0.64 mmol) using Method C. The reaction mixture was stirred for 5 hours after which TLC analysis indicated full conversion of the starting material. Purification by flash column chromatography (hexane-EtOAc, 1:2) gave **15** as a colourless solid (0.120 g, 90%); mp 85.6-86.2; R_{f} = 0.30 (hexane-EtOAc, 1 :2); ¹H NMR (400 MHz, CDCl₃): δ 7.09 (s, 1 H), 7.00 (d, *J* = 8.44, 2 H), 6.81 (d, *J* = 8.44, 2 H), 4.53 (t, *J* = 7.13, 2 H), 4.49-4.33 (m, 1 H), 4.06-3.95 (m, 1 H), 3.78 (s, 3 H), 3.34-3.20 (m, 1 H), 3.26 (s, 3 H), 3.14 (t, *J* = 7.25, 2 H), 2.92-2.73 (m, 2 H), 2.71-2.50 (m, 1 H), 2.08-1.97 (m, 1 H), 1.90-1.74 (m, 1 H), 1.47 (s, 9 H); ¹³C NMR (101 MHz, CDCl₃): δ 158.92, 154.94, 148.43, 130.00, 129.47, 121.94, 114.44, 80.09, 78.85, 57.54, 55.55, 52.01, 46.60, 44*, 40.02, 36.22, 30.29, 28.73; HRMS (ESI): *m*/*z* [M + Na]⁺ calcd for C₂₂H₃₂N₄O₄Na: 439.2321; found: 439.2304.

### (±)-tert-Butyl trans-3-benzyloxy-4-(1-(4-methoxyphenethyl)-1H-1,2,3-triazol-4-yl)piperidine-1-carboxylate (16)

The title compound was prepared from alcohol **4** (98 mg, 0.24 mmol) and benzylbromide (57 µL, 0.48 mmol) using Method C. The reaction mixture was stirred for 22 hours after which TLC analysis indicated full conversion of the starting material. Purification by flash column chromatography (hexane-EtOAc, 1:1) gave **16** as a colourless oil (0.106 g, 89%); R_{f} = 0.31 (hexane-EtOAc, 1:1); ¹H NMR (400 MHz, CDCl₃): δ 7.32-7.21 (m, 3 H), 7.16-7.07 (m, 2 H), 7.01 (s, 1 H), 6.94 (d, *J* = 8.55, 2 H), 6.79 (d, *J* = 8.55, 2H), 4.61-4.38 (m, 4 H), 4.30 (d, *J* = 11.51, 1 H), 4.18-4.02 (m, 1 H), 3.74 (s, 3 H), 3.47-3.35 (m, 1 H), 3.09 (t, *J* = 7.15, 2 H), 3.05-2.95 (m, 1 H), 2.90-2.76 (m, 1 H), 2.2.74-2.61 (m, 1 H), 2.08-1.94 (m, 1 H), 1.77.160 (m, 1 H), 1.47 (s, 9 H); ¹³C NMR (101 MHz, CDCl₃): δ 159.15, 155.03, 148.61, 138.74, 130.01, 129.58, 128.66, 128.12, 127.98, 122.01, 114.67, 80.15, 77.56, 72.11, 55.63, 52.02, 47*, 43*, 40.33, 36.30, 30.45, 28.84; HRMS (ESI): *m*/*z* [M + H]⁺ calcd for C₂₈H₃₇N₄O₄: 493.2815; found: 493.2813.

### (±)-tert-Butyl trans-3-(naphthalen-2-ylmethoxy)-4-(1-phenyl-1H-1,2,3-triazol-4-yl)piperidine-1-carboxylate (17)

The title compound was prepared from alcohol **5** (80 mg, 0.23 mmol) and 2-(bromomethyl)naphthalene (0.101 g, 0.46 mmol) using Method C. The reaction mixture was stirred for 5 hours after which TLC analysis indicated full conversion of the starting material. Purification by flash column chromatography (hexane-EtOAc, 2:1) gave **17** as a colourless oil (98 mg, 88%); R_{f} = 0.31 (hexane-EtOAc, 2:1); ¹H NMR (400 MHz, CDCl₃) δ 7.76 - 7.64 (m, 3 H), 7.61 (d, *J* = 11.4, 2 H), 7.52 (d, *J* = 7.2, 2 H), 7.46-7.33 (m, 5 H), 7.24 (d, *J* = 7.2, 1 H), 4.80 (d, *J* = 11.4, 1 H), 4.63-4.41 (m, 2 H), 4.18-4.06 (m, 1 H), 3.70-3.59 (m, 1 H), 3.08 - 2.96 (m, 1 H), 2.95-2.73 (m, 2 H), 2.19-2.08 (m, 1 H), 2.04-1.87 (m, 1 H), 1.47 (s, 9 H); ¹³C NMR (101 MHz, CDCl₃) δ 155.02, 149.50, 137.59, 136.00, 133.63, 133.41, 129.95, 128.68, 128.46, 128.23, 128.00, 127.05, 126.45, 126.32, 126.28, 120.61, 119.96, 80.23, 77.63, 72.29, 47.69, 43.87, 40.63, 30.49, 28.81; HRMS (ESI): *m*/*z* [M + H]⁺ calcd for C₂₉H₃₃N₄O₄: 485.2553; found: 485.2552.

### (±)tert-Butyl trans-3-(naphthalen-2-ylmethoxy)-4-(1-(4-(N-(naphthalen-2-ylmethyl)acetamido)phenethyl)-1H-1,2,3-triazol-4-yl)piperidine-1-carboxylate (18)

The title compound was prepared from alcohol **6** (43 mg, 0.10 mmol) and 2-(bromomethyl) naphthalene (44 mg, 0.20 mmol) following the general procedure for the formation of ether linkages. Purification by flash column chromatography (hexane-EtOAc, 1:7) gave the title compound as a yellow oil (30 mg, 42%), R_{f} = 0.26 (Hexane-EtOAc, 1 :7); ¹H NMR (400 MHz, CDCl₃) δ 7.82 - 7.67 (m, 6 H), 7.58 (s, 1 H), 7.53 (s, 1 H), 7.45 - 7.37 (m, 4 H), 7.34 (d, *J* = 8.4, 1 H), 7.24 - 7.19 (m, 1 H), 6.95 - 6.88 (m, 3 H), 6.85 - 6.77 (m, 1 H), 5.31 - 4.85 (m, 2 H), 4.78 - 4.69 (m, 1 H), 4.50 - 4.28 (m, 4 H), 4.13 - 3.97 (m, 1 H), 3.66 - 3.54 (m, 1 H), 3.02 (t, *J* = 7.2, 2 H), 2.93 - 2.72 (m, 3 H), 2.05 - 1.95 (m, 1 H), 1.92 - 1.76 (m, 4 H), 1.45 (s, 9H); ¹³C NMR (101 MHz, CDCl₃) δ 170.69, 148.70, 148*, 141.97, 137.22, 135.96, 135.30, 133.56, 133.48, 133.22, 133.07, 130.07, 128.79, 128.55, 128.33, 128.18, 128.13, 128.00, 127.97, 127.83, 127.11, 126.86, 126.53, 126.42, 126.33, 126.23, 121.78, 80.25, 77.56, 53.10, 51.24, 48*, 44*, 40.39, 36.47, 30.64, 28.75, 23.07; HRMS (ESI): *m*/*z* [M + H - NBoc]⁺ calcd for C₃₉H₄₀N₅O₂: 610.3182; found: 610.3177.

### (±) tert-butyl trans-3-(naphthalen-2-ylmethoxy)-4-(1-(3-(N-(naphthalen-2-ylmethyl)acetamido)phenethyl)-1H-1,2,3-triazol-5-yl)piperidine-1-carboxylate (19)

The title compound was prepared from alcohol **7** (20 mg, 0.035 mmol) and 2-(bromomethyl) naphthalene (23 mg, 0.11 mmol) following the general procedure for the formation of ether linkages. Purification by flash column chromatography (hexane-EtOAc, 1 :9) gave the title compound as a colourless oil (18 mg, 72%); R_{f} = 0.26 (hexane-EtOAc, 1:9); ¹H NMR (400 MHz, CDCl₃) δ 7.88 - 7.63 (m, 6 H), 7.52 (s, 1 H), 7.50 - 7.39 (m, 3 H), 7.38 - 7.31 (m, 3 H), 7.11 (t, *J* = 7.8, 1 H), 6.97 - 6.90 (m, 1 H), 6.86 (d, *J* = 7.5, 1 H), 6.81 (d, *J* = 7.8, 1 H), 6.60 (s, 1 H), 5.00 (d, *J* = 14.6, 1 H), 4.89 (d, *J* = 14.6, 1 H), 4.66 - 4.47 (m, 2 H), 4.45 - 4.34 (m, 2 H), 4.29 (d, *J* = 11.6, 1 H), 4.05 - 3.98 (m, 1 H), 3.22 - 3.08 (m, 2 H), 3.07 - 2.96 (m, 1 H), 2.69 - 2.33 (m, 3 H), 1.82 (s, 3 H), 1.65 - 1.52 (m, 1 H), 1.49 - 1.37 (m, 10 H). ¹³C NMR (101 MHz, CDCl₃) δ 170.60, 154.54, 147*, 143.51, 139.63, 135.30, 134.80, 133.54, 133.35, 133.04, 130.91, 129.98, 128.85, 128.71, 128.51, 128.22, 128.08, 128.02, 127.97, 127.72, 127.67, 126.98, 126.68, 126.54, 126.40, 126.19, 125.76, 122.54, 121.08, 80.70, 79.00, 72.25, 49.24, 49*, 45*, 38.75, 36.37, 30*, 28.70, 23.13.

### (±)-tert-Butyl trans-3-(naphthalene-2-ylmethoxy)-4-(1-phenethyl-1H-1,2,3-triazol-5-yl)piperidine-1-carboxylate (20)

The title compound was prepared from alcohol **8** (60 mg, 0.16 mmol) and 2-(bromomethyl)naphthalene (71 mg, 0.32 mmol) using Method C. The reaction mixture was stirred for 19 hours after which TLC analysis indicated full conversion of the starting material. Purification by flash column chromatography (hexane-EtOAc, 1:1) gave **20** as a colourless solid (45 mg, 55%); mp 108.8-112.6; R_{f} = 0.17 (hexane-EtOAc, 1:1); ¹H NMR (400 MHz, CDCl₃): δ 7.81-7.69 (m, 3 H), 7.49-7.41 (m, 2 H), 7.37 (s, 1 H), 7.34 (s, 1 H), 7.23-7.14 (m, 3 H), 6.97 (dd, *J* = 7.03, *J* = 8.30, 3 H), 4.66-4.37 (m, 4 H), 4.31 (d, *J* = 8, 1 H), 4.06-3.94 (m, 1 H), 3.20-3.07 (m, 3 H), 2.53-2.36 (m, 2 H), 2.25-2.13 (m, 1 H), 1.59-1.49 (m, 1 H), 1.42 (s, 9 H), 1.31-1.20 (m, 1 H); ¹³C NMR (101 MHz, CDCl₃): δ 154.71, 139.64, 138.37, 135.12, 133.58, 133.51, 130.74, 129.34, 129.04, 128.70, 128.32, 128.05, 127.21, 126.91, 126.61, 126.45, 125.80, 80.57, 79.31, 72.44, 49.90, 48*, 44*, 38.90, 37.29, 30.28, 28.75; HRMS (ESI): *mlz* [M + Na]⁺ calcd for C₃₁H₃₆N₄O₃Na: 535.2685; found *m*/*z* 535.2670

### (±)-tert-Butyl trans-4-(1-(4-methoxy-phenethyl)-1H-1,2,3-triazol-5-yl)-3-(naphthalene-2-ylmethoxy)piperidine-1-carboxylate (21)

The title compound was prepared from alcohol **9** (0.116 g, 0.29 mmol) and 2-(bromomethyl)naphthalene (0.128 g, 0.58 mmol) using Method C. The reaction mixture was stirred for 18 hours after which TLC analysis indicated full conversion of the starting material. Purification by flash column chromatography (hexane-EtOAc, 1:1) gave the title compound as colourless oil (0.141 g, 89%); R_{f} = 0.35 (hexane-EtOAc, 1:1); ¹H NMR (400 MHz, CDCl₃) δ 7.84 - 7.69 (m, 3 H), 7.50 - 7.42 (m, 2 H), 7.39 - 7.33 (m, 2 H), 7.01- 6.92 (m, 1 H), 6.83 (d, *J* = 8.2, 2 H), 6.73 (d, *J* = 8.4, 2 H), 4.67 - 4.52 (m, 2 H), 4.52 - 4.40 (m, 2 H), 4.35 - 4.27 (m, 1 H), 4.10 - 3.96 (m, 1 H), 3.73 (s, 3 H), 3.18 - 3.01 (m, 3 H), 2.53 - 2.37 (m, 2 H), 2.24 - 2.14 (m, 1 H), 1.62 - 1.49 (m, 1 H), 1.44 (s, 9 H), 1.34 - 1.22 (m, 1 H); ¹³C NMR (101 MHz, CDCl₃) δ 158.82, 154.58, 139.57, 134.95, 133.35, 133.30, 130.68, 130.28, 130.14, 128.64, 128.23, 127.98, 126.83, 126.56, 126.40, 125.72, 114.31, 80.55, 79.03, 72.24, 55.60, 50.11, 48*, 44*, 38.65, 36.33, 30.20, 28.65; HRMS (ESI): *m*/*z* [M + H]⁺ calcd for C₃₂H₃₉N₄O₄: 543.2971 ; found: 543.2973.

### (±) tert-Butyl trans-4-(1-(4-acetamidophenethyl)-1H-1,2,3-triazol-5-yl)-3-(naphthalen-2-ylmethoxy)piperidine-1-carboxylate (22)

The title compound was prepared from alcohol **10** (60 mg, 0.14 mmol) and 2-(bromomethyl) naphthalene (62 mg, 0.28 mmol) following the general procedure for the formation of ether linkages. Purification by flash column chromatography (hexane-EtOAc, 1 :6) gave the title compound as a colourless oil (70 mg, 71%); R_{f} = 0.27 (hexane-EtOAc, 1:6); ¹H NMR (400 MHz, CDCl₃) δ 7.88 - 7.63 (m, 6 H), 7.53 (s, 1 H), 7.50 - 7.32 (m, 7 H), 6.99 - 6.91 (m, 1 H), 6.85 (d, *J* = 7.6, 2 H), 6.79 (d, *J* = 7.6, 2 H), 4.97 (s, 2 H), 4.74 - 4.56 (m, 2 H), 4.55 - 4.40 (m, 2 H), 4.34 (d, *J* = 11.6, 1 H), 4.20 - 4.00 (m, 1 H), 3.34 - 2.98 (m, 3 H), 2.70 - 2.30 (m, 3H), 1.85 (s, 3 H), 1.70 - 1.53 (m, 2 H), 1.44 (s, 9 H); ¹³C NMR (101 MHz, CDCl₃) δ 170.74, 154.58, 150*, 141.89, 139.13, 137*, 135.32, 134.79, 133.55, 133.35, 133.06, 130.99, 130.20, 128.71, 128.56, 128.20, 128.12, 127.98, 127.62, 127.03, 126.95, 126.65, 126.52, 126.40, 126.19, 125.82, 80.75, 78.96, 72.27, 53.20, 49.20, 48*, 44*, 38.87, 36.06, 30*, 28.70, 23.08; HRMS (ESI): *m*/*z* [M + H]⁺ calcd for C₄₄H₄₈N₅0₄: 710.3706; found: 710.3711.

### General procedure for the formation of ester linkages - Method D

To a solution an alcohol (0.5 mmol) in dry CH₂Cl₂ (5 mL) were added a carboxylic acid (0.75 mmol), DMAP (0.9 mmol) and DCC (0.9 mmol). The reaction mixture was stirred at room temperature until TLC analysis indicated full conversion of the alcohol. The reaction mixture was diluted with Et₂O (25 mL) and washed with 5% citric acid (25 mL) and a saturated aqueous solution of NaCl (25 mL) before it was dried over MgSO₄. After removal of the drying agent by filtration, the solvent was removed under reduced pressure. The crude product was purified by flash column chromatography.

### (±)-tert-Butyl trans-3-(2-naphthoyloxy)-4-(1-(4-methoxyphenethyl)-1H-1,2,3-triazol-4-yl)piperidine-1-carboxylate (23)

The title compound was prepared from alcohol **4** (90 mg, 0.40 mmol) and 2-naphthoic acid (57 mg, 0.33 mmol) using Method D. The reaction mixture was stirred for 20 hours after which TLC analysis indicated full conversion of the starting material. Purification by flash column chromatography (hexane-EtOAc, 1:1) gave **23** as a colourless oil (96 mg (77%); R_{f} = 0.22 (hexane-EtOAc, 1:1); ¹H NMR (400 MHz, CDCl₃): δ 8.54 (s, 1 H), 7.99 (d, *J* = 8.68, 1 H), 7.93 (d, *J* = 8.18, 1 H), 7.88-7.81 (m, 2 H), 7.62-7.49 (m, 2 H), 7.10 (s, 1 H), 6.86 (d, *J* = 8.18, 2 H), 6.69 (d, *J* = 8.18, 2 H), 5.25-5.15 (m 1 H), 4.45 (t, *J* = 7.12, 2 H), 4.26-4.14 (m, 1 H), 3.98-3.87 (m, 1 H), 3.70 (s, 3 H), 3.40-3.15 (m, 3 H), 3.03 (t, *J* = 7.24, 2 H), 2.27-2.17 (m, 1 H), 1.91-1.78 (m, 1 H), 1.44 (s, 9 H); ¹³C NMR (101 MHz, CDCl₃): δ 166.05, 158.92, 155.04, 147.81, 135.95, 132.77, 131.58, 129.90, 129.74, 129.24, 128.72, 128.54, 128.10, 127.44, 127.06, 125.56, 121.17, 114.44, 80.42, 71.87, 55.49, 52.21, 47*, 43*, 38.46, 36.20, 29*, 28.70; HRMS (ESI): *m*/*z* [M + Na]⁺ calcd for C₃₂H₃₆N₄O₅Na: 579.2583; found: 579.2580.

### (±)-tert-Butyl trans-3-(2-benzoyloxy)-4-(1-(4-methoxyphenethyl)-1H-1,2,3-triazol-4-yl)piperidine-1-carboxylate (24)

The title compound was prepared from alcohol **4** (98 mg, 0.24 mmol) and benzoic acid (46 mg, 0.38 mmol) using Method D. The reaction mixture was stirred for 21 hours after which TLC analysis indicated full conversion of the starting material. Purification by flash column chromatography (hexane-EtOAc, 1:2) gave **24** as a colourless oil (95 mg, 75%); R_{f} = 0.35 (hexane-EtOAc, 1:2); ¹H NMR (400 MHz, CDCl₃) δ 8.12 (d, *J* = 7.3, 1 H), 7.98 (d, *J* = 7.4, 2 H), 7.51 - 7.37 (m, 2 H), 7.09 (s, 1 H), 6.89 (d, *J* = 8.4, 2 H), 6.73 (d, *J* = 8.5, 2 H), 5.19 - 5.06 (m, 1 H), 4.47 (t, *J* = 7.0, 2 H), 4.26 - 4.00 (m, 1 H), 3.95 - 3.84 (m, 1 H), 3.75 (s, 3 H), 3.38-3.11 (m, 3 H), 3.05 (t, *J* = 6.9, 2 H), 2.24 - 2.13 (m, 1 H), 1.87 - 1.74 (m, 1 H), 1.43 (s, 9 H); ¹³C NMR (101 MHz, CDCl₃) δ 165.84, 158.93, 155.01, 147.72, 133.47, 130.02, 129.92, 129.24, 128.74, 128.71, 121.21, 114.46, 80.41, 77.56, 71.71, 55.56, 52.24, 46.59, 38.33, 36.21, 30.02, 28.66; HRMS (ESI): *m*/*z* [M + H]⁺ calcd for C₂₈H₃₅N₄O₅: 507.2608; found; 507.2600.

### (±)-tert-Butyl trans-3-(2-naphthoyloxy)-4-(1-phenyl-1H-1,2,3-triazol-4-yl)piperidine-1-carboxylate (25)

The title compound was prepared from alcohol **5** (73 mg, 0.21 mmol) and 2-naphthoic acid (55 mg, 0.32 mmol) using Method D. The reaction mixture was stirred for 20 hours after which TLC analysis indicated full conversion of the starting material. Purification by flash column chromatography (hexane-EtOAc, 2:1) gave **25** as a colourless solid (75 mg, 72%); mp 174.9-177.1; R_{f} = 0.25 (hexane-EtOAc, 2:1); ¹H NMR (400 MHz, CDCl₃): δ 8.56 (s, 1 H), 8.01 (d, *J* = 8.5, 1 H), 7.92 (d, *J* = 7.88, 1 H), 7.87-7.80 (m, 3 H), 7.62 (d, *J* = 7.89, 2 H), 7.59-7.48 (m, 2 H), 7.47-4.40 (m, 2 H), 7.39-7.33 (m, 1 H), 5.41-5.29 (m, 1 H), 4.38-4.25 (m, 1 H), 4.12-3.99 (m, 1 H), 3.55-3.43 (m, 1 H), 3.40-3.20 (m, 2 H), 2.39-2.28 (m, 1 H), 2.07-1.89 (m, 1 H), 1.46 (s, 9 H); ¹³C NMR (101 MHz, CDCl₃): δ 166.14, 155.02, 148.84, 137.34, 135.96, 132.76, 131.63, 130.01, 129.72, 128.98, 128.73, 128.56, 128.09, 127.05, 125.53, 120.79, 119.15, 80.49, 71.80, 49.50, 48*, 42*, 38.66, 30*, 28.72; HRMS (ESI): *m*/*z* [M + Na]⁺ calcd for C₂₉H₃₀N₄NaO₄: 521.2165; found: 512.2167.

### (±)-tert-Butyl trans-3-(2-benzoyloxy)-4-(1-phenyl-1H-1,2,3-triazol-4-yl)piperidine-1-carboxylate (26)

The title compound was prepared from alcohol **5** (0.105 g, 0.30 mmol) and benzoic acid (56 mg, 0.46 mmol) Method D. The reaction mixture was stirred for 18 hours after which TLC analysis indicated full conversion of the starting material. Purification by flash column chromatography (hexane-EtOAc, 2:1) gave the **26** as a colourless solid (0.130 g, 96%); mp 155.3-157.4; R_{f} = 0.34 (hexane-EtOAc, 2:1); ¹H NMR (400 MHz, CDCl₃) δ 8.01 (d, *J* = 7.6, 2 H), 7.83 (s, 1 H), 7.64 (d, *J* = 7.8, 2 H), 7.54 (t, *J* = 7.3, 1 H), 7.48 (t, *J* = 7.7, 2 H), 7.44-7-36 (m, 3 H), 5.34-523 (m, 1 H), 4.19-4.15 (m, 1 H), 4.07-3.96 (m, 1 H), 3.49-3.37 (m, 1 H), 3.35-3.12 (m, 2 H), 2.36-2.23 (m, 1 H), 2.03-1.88 (m, 1 H), 1.45 (s, 9 H); ¹³C NMR (101 MHz, CDCl₃) δ 165.92, 154.98, 148.77, 137.32, 133.51, 130.12, 130.03, 130.01, 128.98, 128.72, 120.76, 119.14, 80.45, 71.62, 46.73, 42.41, 38.49, 30.01, 28.67; HRMS (ESI): *m*/*z* [M + Na]⁺ calcd for C₂₅H₂₈N₄O₄Na: 471.2008; found: 471.2004.

### General procedure for Boc deprotection - Method E

The Boc-protected piperidine derivative was dissolved in a mixture of CH₂Cl₂ and TFA (1:1, 2 mL/mmol) and the reaction mixture was stirred at room temperature for 30 min after which all starting material was consumed. The solvents were removed under reduced pressure and the residue was coevaporated with toluene (2 x 25 mL), CH₃CN (2 x 25 mL) and CH₂Cl₂ (2 x 25 mL) to afford the amine as its TFA salt. The crude product was purified by semi-preparative HPLC using a C18-column. Fractions of equal purity were pooled and concentrated under vacuum before the aqueous solution was lyophilized. All final compound were found to be of higher than 95% purity (HPLC).

### (±)-trans-3-(Naphthalene-2-ylmethoxy)-4-(1-phenethyl-1H-1,2,3-triazol-4-yl)piperidine (27)

The title compound was prepared from Boc-protected piperidine **11** (50 mg, 0.1 mmol) using Method E. Purification by semi-preparative RP-HPLC afforded **27** as a colourless powder (16 mg, 40%); ¹H NMR (400 MHz, CDCl₃): δ 7.87-7.79 (m, 3 H), 7.71 (s, 1 H), 7.52 (s, 1 H), 7.51-7.45 (m, 2 H), 7.36-7.30 (m, 1 H), 7.21-7.09 (m, 3 H), 7.05 (d, *J* = 6.94, 2 H), 4.70 (d, *J* = 9.83, 1 H), 4.65-4.47 (m, 3 H), 3.94-3.85 (m, 1 H), 3.52-3.42 (m, 1 H), 3.35-3.28 (m, 1 H), 3.26-3.18 (m, 1 H), 3.17-3.04 (m, 4 H), 2.33-2.21 (m, 1 H), 2.09-1.96 (m, 1 H); ¹³C NMR (101 MHz, CDCl₃): δ 148.38, 139.52, 137.16, 135.52, 135.45, 130.63, 130.48, 130.12, 129.80, 129.59, 128.88, 128.76, 128.22, 128.12, 127.81, 125.30, 75.47, 73.98, 53.41, 47.32, 44.17, 38.22, 37.71, 27.17; HRMS (ESI): *m*/*z* [M + H]⁺ calcd for C₂₆H₂₉N₄O: 413.2341; found: 413.2339.

### (±)-trans-3-Methoxy-4-(1-phenethyl-1H-1,2,3-triazol-4-yl)piperidine (28)

The title compound was prepared from Boc-protected piperidine **12** (96 mg, 0.25 mmol) using Method E. Purification by semi-preparative RP-HPLC afforded **28** as a colourless powder (37 mg, 53%); ¹H NMR (400 MHz, MeOD): δ 7.65 (s, 1 H), 7.28-7.19 (m, 3 H), 7.11 (d, *J* = 7.19, 2 H), 4.65 (t, *J* = 7.13, 2 H), 3.70-3.63 (m, 1 H), 3.39-3.28 (m, 2 H) 3.37 (s, 3 H), 3.43-3.24 (m, 1 H), 3.20 (t, *J* = 7.04, 2 H), 3.15-3.05 (m, 2 H), 2.34-2.23 (m, 1 H), 2.06-1.96 (m, 1 H); ¹³C NMR (101 MHz, MeOD): δ 148.27, 139.62, 130.70, 130.52, 128.80, 125.33, 77.28, 58.73, 53.48, 46.24, 43.77, 38.29, 36.49, 26.26; HRMS (ESI): *m*/*z* [M + H]⁺ calcd for C₁₆H₂₁N₄O: 287.1872; found: 287.1870.

### (±)-trans-3-(Benzyloxy)-4-(1-phenethyl-1H-1,2,3-triazol-4-yl)piperidine (29)

The title compound was prepared from Boc-protected piperidine **13** (52 mg, 0.11 mmol) using Method E. Purification by semi-preparative RP-HPLC afforded 29 as a colourless oil (34 mg, 65%); ¹H NMR (400 MHz, CDCl₃): δ 7.58 (s, 1 H), 7.34-7.27 (m, 3 H), 7.25-7.14 (m, 5 H), 7.09 (d, *J* = 6.63, 2 H), 4.67-4.56 (m, 2 H), 4.53 (d, *J* = 11.81, 1 H), 4.45 (d, *J* = 11.81, 1 H), 3.90-3.83 (m, 1 H), 3.49-3.38 (m, 1 H), 3.34-3.25 (m, 1 H), 3.24-3.15 (m, 3 H), 3.15-3-02 (m, 2 H), 2.33-2.21 (m, 1 H), 2.08-1.96 (m, 1 H); ¹³C NMR (101 MHz, CDCl₃): δ 148.36, 139.76, 139.57, 130.67, 130.52, 130.42, 130.34, 129.98, 128.79, 125.32, 75.47, 73.92, 53.47, 47.22, 44.06, 38.28, 37.51, 27.01; HRMS (ESI): *m*/*z* [M + H]⁺ calcd for C₂₂H₂₇N₄O: 363.2185; found: 363.2184.

### (±)-trans-3-Hydroxy-4-(1-(4-methoxy-phenethyl)-1H-1,2,3-triazol-4-yl)piperidine (30)

The title compound was prepared from Boc-protected piperidine **4** (90 mg, 0.22 mmol) using Method E. Purification by semi-preparative RP-HPLC afforded 30 as a colourless solid (69 mg, 75%); ¹H NMR (400 MHz, MeOD): δ 7.66 (s, 1 H), 7.02 (d, *J* = 8.50, 2 H), 6.81 (d, *J* = 8.50, 2 H), 4.59 (t, *J* = 6.88, 2 H), 4.02-3.93 (m, 1 H), 3.74 (s, 3 H), 3.44-3.28 (m, 4 H), 3.18-3.05 (m, 3 H), 3.04-2.89 (m, 2 H), 2.35-2.24 (m, 1 H), 2.06-1.93 (m, 1 H); ¹³C NMR (101 MHz, MeOD): δ 161.00, 148.68, 131.68, 131.46, 125.28, 115.91, 68.31, 50*, 56.51, 53.77, 44.55, 40.12, 37.52, 27.42; HRMS (ESI): *m*/*z* [M + H]⁺ calcd for C₁₆H₂₃N₄O₂: 303.1821; found: 303.1819.

### (±)-trans-4-(1-(4-Methoxyphenethyl)-1H-1,2,3-triazol-4-yl)-3-(naphthalen-2-ylmethoxy)piperidine (31)

The title compound was prepared from Boc-protected piperidine **14** (0.100 g, 0.18 mmol) using Method E. Purification by semi-preparative RP-HPLC afforded **31** as a colourless solid (62 mg, 72%); ¹H NMR (400 MHz, DMSO): δ 7.98-7.86 (m, 4 H), 7.74 (s, 1 H), 7.60-7.51 (m, 2 H), 7.34 (d, *J* = 7.98, 1 H), 7.08 (d, *J* = 7.98, 2 H), 6.86 (d, *J* = 7.98, 2 H), 4.70 (d, *J* = 12.11, 1 H), 4.64-4.51 (m, 3 H), 3.96-3.86 (m, 1 H), 3.67 (s, 3 H), 3.55-3.47 (m, 1 H), 3.30-3.16 (m, 3 H), 3.14-2.99 (m, 4 H), 2.20-2.09 (m, 1 H), 2.04-1.90 (m, 1 H); ¹³C NMR (101 MHz, DMSO): δ 158.83, 147.13, 136.40, 133.58, 133.38, 130.55, 130.33, 128.69, 128.58, 128.48, 127.14, 126.98, 126.91, 126.69, 123.48, 114.66, 74.66, 71.87, 55.82, 51.59, 45.74, 42.76, 36.91, 35.89, 26.88; HRMS (ESI): *m*/*z* [M + H]⁺ calcd for C₂₇H₃₀N₄O₂: 443.2447; found: 443.2438.

### (±)-trans-3-Methoxy-4-(1-(4-methoxy-phenethyl)-1H-1,2,3-triazol-4-yl)piperidine (32)

The title compound was prepared from Boc-protected piperidine 15 (45 mg, 0.11 mmol) using Method E. Purification by semi-preparative RP-HPLC afforded 32 as a colourless solid (39 mg, 82%); ¹H NMR (400 MHz, MeOD): δ 7.66 (s, 1 H), 7.01 (d, *J* = 8.84, 2 H), 6.80 (d, *J* = 8.84, 2 H), 4.60 (t, *J* = 7.01, 2 H), 3.73 (s, 3 H), 3.71-3.63 (m, 1 H), 3.47-3-39 (m, 1 H), 3.36-3.26 (m, 4 H), 3.24-3.01 (m, 5 H), 2.33-2-22 (m, 1 H), 2.07-1.95 (m, 1 H); ¹³C NMR (101 MHz, MeOD): δ 160.97, 148.36, 131.68, 131.45, 125.26, 115.90, 77.38, 58.75, 56.51, 53.70, 46.31, 43.84, 37.45, 36.80, 26.49; HRMS (ESI): *m*/*z* [M + H]⁺ calcd for C₁₇H₂₅N₄O₂: 317.1978; found: 317.1977.

### (±)-trans-3-Benzyloxy-4-(1-(4-methoxy-phenethyl)-1H-1,2,3-triazol-4-yl)piperidine (33)

The title compound was prepared from Boc-protected piperidine **16** (0.100 mg, 0.20 mmol) using Method E. Purification by semi-preparative RP-HPLC afforded **33** as a colourless solid (50 mg, 50%); ¹H NMR (400 MHz, MeOD): δ 7.33-7.24 (m, 3 H), 7.21-7.13 (m, 2 H), 7.07 (s, 1 H), 6.99 (d, *J* = 8.58, 2 H), 6.80 (d, *J* = 8.58, 2 H), 4.57-4.43 (m, 3 H), 4.36 (d, *J* = 11.59, 1 H), 3.99-3.89 (m, 1 H), 3.75 (s, 3 H), 3.60-3.49 (m, 1 H), 3.43-3.30 (m, 1 H), 3.20-2.93 (m, 5 H), 2.40-2.29 (m, 1 H), 2.14-1.99 (m, 1 H); ¹³C NMR (101 MHz, MeOD): δ 159.08, 146*, 137.46, 129.98, 129.08, 128.83, 128.41, 128.28, 122.19, 114.57, 73.77, 72.42, 55.57, 52.31, 45.84, 42.67, 36.67, 36.09, 25.97; HRMS (ESI): *m*/*z* [M + H]⁺ calcd for C₂₃H₂₉N₄O₂: 393.2291; found: 393.2283.

### (±)-trans-3-(Naphthalen-2-ylmethoxy)-4-(1-phenyl-1H-1,2,3-triazol-4-yl)piperidine (34)

The title compound was prepared from Boc-protected piperidine **17** (47 mg, 0.097 mmol) using Method E. Purification by semi-preparative RP-HPLC afforded **34** as a colourless solid (28 mg, 57%); ¹H NMR (400 MHz, DMSO) δ 8.97 (s, 1 H), 8.80 (s, 1 H), 8.68 (s, 1 H), 7.89 - 7.82 (m, 3 H), 7.82 - 7.74 (m, 2 H), 7.70 (s, 1 H), 7.60 (t, *J* = 7.8, 2 H), 7.53 - 7.43 (m, 3 H), 7.33 - 7.26 (m, 1 H), 4.78 (d, *J* = 12.2, 1 H), 4.63 (d, *J* = 12.2, 1 H), 4.04 - 3.93 (m, 1 H), 3.65 - 3.55 (m, 1 H), 3.35 - 3.23 (m, 2 H), 3.16 - 3.01 (m, 2 H), 2.26 - 2.16 (m, 1 H), 2.14 - 2.01 (m, 1 H); ¹³C NMR (101 MHz, CDCl₃) δ 148.50, 137.60, 136.40, 133.55, 133.35, 130.82, 129.49, 128.67, 128.54, 128.47, 127.12, 127.06, 126.92, 126.70, 121.98, 120.78, 74.64, 71.81, 45.72, 42.75, 37.11, 26.73; HRMS (ESI): *m*/*z* [M + H]⁺ calcd for C₂₄H₂₅N₄O: 385.2028; found: 385.2021.

### (±) trans-N-(4-(2-(4-(3-(Naphthalen-2-ylmethoxy)piperidin-4-yl)-1H-1,2,3-triazol-1-yl)ethyl)phenyl))-N-(naphthalene-2-ylmethyl)acetamide (35)

The title compound was synthesized from compound **18** (30 mg, 0.042 mmol) using Method E. Purification by preparative HPLC and lyophilisation gave the TFA salt of title compound as a colourless solid (13.3 mg, 52%); ¹H NMR (400 MHz, MeOD) δ 7.86 - 7.69 (m, 6 H), 7.66 (s, 1 H), 7.57 (s, 1 H), 7.52 - 7.41 (m, 4 H), 7.29 (t, *J* = 9.7, 2 H), 6.99 (d, *J* = 8.0, 2 H), 6.83 (d, *J* = 8.0, 2 H), 4.94 (s, 3 H), 4.67 (d, *J* = 12.0, 1 H), 4.62 - 4.48 (m, 3 H), 4.00 - 3.83 (m, 1 H), 3.61 - 3.51 (m, 1 H), 3.21 - 2.97 (m, 5 H), 2.27 - 2.15 (m, 1 H), 2.06 - 1.94 (m, 1 H), 1.79 (s, 3 H); HRMS (ESI): *m*/*z* [M + H]⁺ calcd for C₃₉H₄₀N₅O₂: 610.3182; found 610.3187

### (±) trans-N-(3-(2-(4-(3-(Naphthalen-2-ylmethoxy)piperidin-4-yl)-1H-1,2,3-triazol-1-yl) ethyl)phenyl)-N-(naphthalene-2-ylmethyl)acetamide (36)

The title compound was synthesized from compound **19** (60 mg, 0.085 mmol) following the general procedure for the *N-*Boc-deprotection using TFA. Purification by preparative HPLC and lyophilisation gave the TFA salt of the title compound as a colourless solid (32 mg, 52%); ¹H NMR (400 MHz, MeOD) δ 7.96 - 7.75 (m, 5 H), 7.74 - 7.71 (m, 1 H), 7.68 (s, 1 H), 7.54 - 7.36 (m, 6 H), 7.32 (t, *J* = 9.7, 2 H), 7.09 (t, *J* = 7.7, 1 H), 6.96 (d, *J* = 7.2, 1 H), 6.83 (d, *J* = 7.5, 1 H), 6.77 (s, 1 H), 5.04 - 4.84 (m, 3 H), 4.69 (d, *J* = 12.1, 1 H), 4.57 (d, *J* = 12.1, 1 H), 4.50 - 4.33 (m, 2 H), 3.91 - 3.80 (m, 1 H), 3.59 - 3.46 (m, 1 H), 3.16 - 2.99 (m, 5 H), 2.26 - 2.12 (m, 1 H), 2.02 - 1.90 (m, 1 H), 1.82 (s, 3 H); ¹³C NMR (101 MHz, MeOD) δ 172.85, 147.64, 143.71, 140.64, 136.31, 135.94, 134.64, 134.63, 134.55, 130.82, 129.76, 129.64, 129.29, 128.93, 128.77, 128.74, 128.67, 127.92, 127.82, 127.67, 127.39, 127.28, 127.06, 126.89, 124.22, 101.40, 74.68, 73.04, 53.73, 52.20, 46.46, 43.34, 36.82, 26.52, 22.79; HRMS (ESI): *m*/*z* [M + H]⁺ calcd for C₃₉H₄₀N₅O₂: 610.3182; found 610.3157.

### (±)-trans-3-(Naphthalen-2-ylmethoxy)-4-(1 -phenethyl-1H-1,2,3-triazol-5-yl)piperidine (37)

The title compound was prepared from Boc-protected piperidine **20** (75 mg, 0.15 mmol) using Method E. Purification by semi-preparative RP-HPLC afforded **37** as a colourless solid (38 mg, 45%); ¹H NMR (400 MHz, CDCl₃): δ 7.88 - 7.82 (m, 1 H), 7.82 - 7.73 (m, 2 H), 7.55 - 7.44 (m, 4 H), 7.28 - 7.17 (m, 3 H), 7.06 - 6.96 (m, 3 H), 4.74 - 4.55 (m, 3 H), 4.43 (d, *J* = 11.9, 1 H), 3.80 - 3.68 (m, 1 H), 3.58 - 3.47 (m, 1 H), 3.37 - 3.31 (m, 1 H), 3.26 - 3.09 (m, 2 H), 2.98 - 2.89 (m, 1 H), 2.89 - 2.78 (m, 1 H), 2.78 - 2.67 (m, 1 H), 1.85 - 1.71 (m, 1 H), 1.57 - 1.46 (m, 1 H); ¹³C NMR (101 MHz, MeOD) δ 140.81, 140.01, 136.44, 135.46, 135.37, 132.09, 131.00, 130.59, 130.29, 129.78, 129.57, 128.95, 128.80, 128.26, 128.21, 127.48, 77.52, 74.11, 51.63, 48.03, 45.16, 38.40, 38.10, 28.31; HRMS (ESI): *m*/*z* [M + Na]⁺ calcd for C₂₆H₂₉N₄O: 413.2341 ; found: 413.2334.

### (±)-trans-4-(1-(4-Methoxyphenethyl)-1H-1 ,2,3-triazol-5-yl)-3-(naphthalen-2-ylmethoxy)piperidine (38)

The title compound was prepared from Boc-protected piperidine **21** (50 mg, 0.092 mmol) using Method E. Purification by semi-preparative RP-HPLC afforded **38** as a colourless solid (30 mg, 74%); ¹H NMR (400 MHz, MeOD) δ 7.84 - 7.79 (m, 1 H), 7.78 - 7.69 (m, 2 H), 7.50 - 7.41 (m, 4 H), 6.99 (d, *J* = 8.4, 1 H), 6.86 (d, *J* = 8.4, 2 H), 6.74 (d, *J* = 8.5, 2 H), 4.67 (d, *J* = 11.9, 1 H), 4.63 - 4.47 (m, 2 H), 4.39 (d, *J* = 11.9, 1 H), 3.77 - 3.66 (m, 4 H), 3.57 - 3.46 (m, 1 H), 3.38 - 3.28 (m, 1 H), 3.15 - 2.97 (m, 2 H), 2.96 - 2.77 (m, 2 H), 2.75 - 2.64 (m, 1 H), 1.86 - 1.72 (m, 1 H), 1.62 - 1.49 (m, 1 H); ¹³C NMR (101 MHz, MeOD) δ 160.12, 139.98, 135.61, 134.59, 134.51, 131.14, 130.94, 129.42, 128.93, 128.70, 128.08, 127.38, 127.34, 126.63, 115.05, 76.63, 73.24, 55.68, 51.06, 47.19, 44.25, 37.23, 36.66, 27.55; HRMS (ESI): *m*/*z* [M + H]⁺ calcd for C₂₇H₃₁N₄O₂: 443.2447; found 443.2435.

### (±) trans-N-(4-(2-(5-(-3-(Naphthalen-2-ylmethoxy)piperidin-4-yl)-1H-1,2,3-triazol-1-yl)ethyl)phenyl)-N-(naphthalen-2-ylmethyl)acetamide (39)

The title compound was synthesized from compound **22** (50 mg, 0.07 mmol) following the general procedure for the *N-*Boc-deprotection using TFA. Purification by preparative HPLC and lyophilisation gave the TFA salt of the title compound as a colourless solid (24 mg, 47%); ¹H NMR (400 MHz, MeOD) δ 7.84 - 7.66 (m, 5 H), 7.65 (d, *J* = 8.6, 1 H), 7.51 - 7.37 ( m, 7 H), 7.35 - 7.23 (m, 1 H), 6.96 (d, *J* = 8.4, 1 H), 6.91 (d, *J* = 7.6, 2 H), 6.83 (d, *J* = 7.9, 2 H), 4.95 (s, 3 H), 4.67 (d, *J* = 11.8, 1 H), 4.51 (t, *J* = 7.4, 2 H), 4.37 (d, *J* = 11.7, 1 H), 3.86 - 3.71 (m, 1 H), 3.65 - 3.54 (m, 1 H), 3.28 - 3.20 (m, 1 H), 3.16 - 3.05 (m, 3 H),3.03 - 2.89 (m, 1 H), 1.85 - 1.73 (m, 5 H); ¹³C NMR (101 MHz, MeOD) δ 172.96, 142.27, 139.74, 139.07, 135.97, 135.57, 134.68, 134.56, 134.48, 134.23, 131.13, 129.41, 129.29, 129.23, 128.91, 128.78, 128.67, 128.26, 128.20, 127.40, 127.37, 127.26, 127.02, 126.81, 76.81, 73.28, 53.78, 50.10, 47.07, 44.19, 37.10, 36.18, 27.87, 22.62; HRMS (ESI): *m*/*z* [M + H]⁺ calcd for C₃₉H₄₀N₅O₂: 610.3182; found 610.3177.

### (±)-trans-4-(1-(4-Methoxyphenethyl)-1H-1,2,3-triazol-4-yl)piperidin-3-yl 2-naphthoate (40)

The title compound was prepared from Boc-protected piperidine 23 (95 mg, 0.18 mmol) using Method E. Purification by semi-preparative RP-HPLC afforded **40** as a colourless solid (50 mg, 40%); ¹H NMR (400 MHz, DMSO) δ 9.18 - 8.95 (m, 2 H), 8.67 (s, 1 H), 8.14 (d, *J* = 8.0, 1 H), 8.11 - 7.98 (m, 4 H), 7.74 - 7.61 (m, 2 H), 6.87 (d, *J* = 8.4, 2 H), 6.60 (d, *J* = 8.4, 2 H), 5.47 - 5.38 (m, 1 H), 4.48 (t, *J* = 7.0, 2 H), 3.63 - 3.54 (m, 4 H), 3.54 - 3.43 (m, 1 H), 3.37 - 3.24 (m, 2 H), 3.24 - 3.12 (m, 1 H), 2.96 (t, *J* = 6.9, 2 H), 2.31 - 2.19 (m, 1 H), 2.19 - 2.04 (m, 1 H); ¹³C NMR (101 MHz, DMSO) δ 165.70, 158.74, 146.34, 136.16, 132.91, 132.00, 130.45, 130.29, 130.26, 129.80, 129.33, 128.72, 128.08, 127.29, 125.87, 123.42, 114.51, 70.03, 55.75, 51.78, 45.16, 42.69, 35.90, 35.83, 26.67; HRMS (ESI): *m*/*z* [M + H]⁺ calcd for C₂₇H₂₉N₄O₃: 457.2240; found 457.2235.

### (±)-trans-4-(1-(4-Methoxyphenethyl)-1H-1,2,3-triazol-4-yl)piperidin-3-yl 2-benzoate (41)

The title compound was prepared from Boc-protected piperidine **24** (95 mg, 0.19 mmol) using Method E. Purification by semi-preparative RP-HPLC afforded **41** as a colourless solid (55 mg, 53%); ¹H NMR (400 MHz, DMSO) δ 9.13 - 8.91 (m, 2 H), 8.04 - 7.95 (m, 3 H), 7.69 (t, *J* = 7.4, 1 H), 7.55 (t, *J* = 7.7, 2 H), 6.91 (d, *J* = 8.5, 2 H), 6.67 (d, *J* = 8.5, 2 H), 5.39 - 5.30 (m, 1 H), 4.49 (t, *J* = 7.0, 2 H), 3.66 (s, 3 H), 3.58 - 3.50 (m, 1 H), 3.45 - 3.37 (m, 1 H), 3.34 - 3.09 (m, 3 H), 2.98 (t, *J* = 7.0, 2 H), 2.26 - 2.15 (m, 1 H), 2.14 - 2.00 (m, 1 H); ¹³C NMR (101 MHz, DMSO): δ 165.57, 158.81, 146.31, 134.69, 130.49, 130.44, 130.32, 129.98, 129.67, 123.38, 114.59, 69.87, 55.87, 51.79, 45.07, 42.63, 35.83, 35.79, 26.63; HRMS (ESI): *m*/*z* [M + H]⁺ calcd for C₂₃H₂₇N₄O₃: 407.2083; found: 407.2077.

### (±)-trans-4-(1-phenyl-1H-1,2,3-triazol-4-yl)piperidin-3-yl 2-naphthoate (42)

The title compound was prepared from Boc-protected piperidine **25** (75 mg, 0.15 mmol) using Method E. Purification by semi-preparative RP-HPLC afforded **42** as a colourless solid (35 mg, 46%); ¹H NMR (400 MHz, DMSO): δ 8.65 (s, 1 H), 8.05 (s, 1 H), 7.97 (s, 1 H), 7.35 (d, *J* = 8.0, 1 H), 7.28 - 7.22 (m, 3 H), 7.06 (d, *J* = 8.0, 2 H), 6.96 - 6.82 (m, 2 H), 6.78 (t, *J* = 7.8, 2 H), 6.68 (t, *J* = 7.4, 1 H), 5.05 - 4.66 (m, 1 H), 2.87 - 2.79 (m, 2 H), 2.65 - 2.40 (m, 3 H), 1.68 - 1.58 (m, 1 H), 1.58 - 1.50 (m, 1 H); ³C NMR (101 MHz, DMSO) δ 147.52, 137.49, 136.14, 132.93, 132.14, 130.80, 130.26, 129.77, 129.57, 129.28, 128.70, 128.03, 127.31, 125.94, 122.14, 120.81, 69.82, 44.86, 42.20, 35.72, 25.84; HRMS (ESI): *m*/*z* [M + H]⁺ calcd for C₂₄H₂₃N₄O₂ [M + H]⁺: 399.1821; found: 399.1820.

### (±)-trans-4-(1-phenyl-1H-1,2,3-triazol-4-yl)piperidin-3-yl 2-benzoate (43)

The title compound was prepared from Boc-protected piperidine **26** (50 mg, 0.11 mmol) using Method E. Purification by semi-preparative RP-HPLC afforded 43 as a colourless solid (28 mg, 55%); ¹H NMR (400 MHz, MeOD) δ 8.50 (s, 1 H), 8.05 (d, *J* = 7.2, 2 H), 7.76 (d, *J* = 7.9, 2 H), 7.63 (t, *J* = 7.4, 1 H), 7.55 (t, *J* = 7.6, 2 H), 7.52 - 7.43 (m, 3 H), 5.61 - 5.51 (m, 1 H), 3.84 - 3.75 (m, 1 H), 3.67 - 3.52 (m, 2 H), 3.43 - 3.24 (m, 2 H), 2.54 - 2.41 (m, 1 H), 2.41 - 2.27 (m, 1 H); ¹³C NMR (101 MHz, MeOD): δ 167.40, 148.73, 139.15, 135.75, 131.83, 131.68, 131.23, 131.02, 130.60, 123.32, 122.37, 71.04, 46.87, 44.30, 37.39, 27.57; HRMS (ESI): *m*/*z* [M + H]⁺ calcd for C₂₀H₂₁N₄O₂: 349.1665 found: 349.1661.

### (±) tert-Butyl trans-4-ethynyl-3-(naphthalen-2-ylmethoxy)piperidine-1-carboxylate (44)

The title compound was prepared from 2 (60 mg, 0.3 mmol) and 2-(bromomethyl)-naphthalene (0.132 g, 0.6 mmol) using Method C. Purification of the crude product by flash column chromatography (hexane-EtOAc, 9:1) gave the title compound as a colourless solid (88 mg, 80%); mp 60.9 - 64.7; R_{f} = 0.30 (hexane-EtOAc, 9:1): 0.30; ¹H NMR (400 MHz, CDCl₃): δ 7.86-7.77 (m, 4 H), 7.52-7.41 (m, 3 H), 4.87 (d, *J* = 12.0, 1 H), 4.77 (d, *J* = 12.0, 1 H), 3.81-3.68 (m, 1 H), 3.61-3.51 (m, 1 H), 3.50-3.40 (m, 2 H), 3.39-3.30 (m, 1 H), 2.77-2.69 (m, 1 H), 2.15 (d, *J* = 2.1, 1 H), 2.10-2.00 (m, 1 H), 1.61-1.50 (m, 1 H), 1.43 (s, 9 H); ¹³C NMR (101 MHz, CDCl₃): δ 155.22, 136.18, 133.76, 133.51, 128.48, 128.26, 128.06, 126.75, 126.41, 126.19, 84.93, 80.04, 75.83, 71.98, 71.37, 45*, 41*, 33.28, 28.79, 28.43; HRMS (ESI): *m*/*z* [M + Na]⁺ calcd for C₂₃H₂₇NO₃Na: 388.1889; found: 388.1870.

### (±) tert-Butyl 4-(1-(3-acetamidophenethyl)-1H-1 ,2,3-triazol-5-yl)-3-(naphthalen-2-ylmethoxy)piperidine-1-carboxylate (45)

The title compound was prepared from alkyne **44** (0.12 g, 0.33 mmol) and *N-*(3-(2-azidoethyl)phenyl)acetamide (74 mg, 0.36 mmol) following the general procedure for Ru(II)-catalyzed cycloaddition. The reaction was stirred at rt for 22 h. Purification by flash column chromatography (hexane-EtOAc, 1 :6) gave the title compound as a colourless oil (0.142 g, 76%), R_{f} = 0.27 (hexane-EtAOc, 1:6); ¹H NMR (400 MHz, CDCl₃) δ 7.88 - 7.64 (m, 3 H), 7.58 - 7.42 (m, 2 H), 7.36 (d, *J* = 7.9, 2 H), 7.14 (t, *J* = 7.8, 2 H), 7.03 - 6.88 (m, 1 H), 6.80 - 6.53 (m, 1 H), 4.67 - 4.42 (m, 4 H), 4.31 (d, *J* = 11.4, 1 H), 4.07 - 3.94 (m, 1 H), 3.18 - 2.95 (m, 3 H), 2.63 - 2.34 (m, 2 H), 2.32 - 2.19 (m, 1 H), 2.13 (s, 3 H), 1.58 - 1.49 (m, 1 H), 1.43 (s, 9 H), 1.33 - 1.19 (m, 1 H); ¹³C NMR (101 MHz, CDCl₃) δ 168.75, 154.63, 139.81, 138.90, 138.76, 135.02, 133.35, 133.28, 132.42, 132.32, 130.60, 129.55, 128.92, 128.80, 128.60, 128.21, 127.98, 126.78, 126.57, 126.41, 125.73, 124.91, 120.47, 118.62, 80.53, 79.09, 72.13, 49.64, 47*, 43*, 38.58, 37.10, 29*, 28.67, 24.88; HRMS (ESI): *m*/*z* [M + Na]⁺ calcd for C₃₃H₃₉N₅NaO₄: 592.2893; found: 592.2900.

### (±) trans-N-(3-(2-(5-(3-(Naphthalen-2-ylmethoxy)piperidin-4-yl)-1H-1 ,2,3-triazol-1-yl) ethyl)phenyl)acetamide (46)

The title compound was synthesized from compound **45** (0.127 g, 0.22 mmol) using Method E. Purification by preparative HPLC and lyophilisation gave the TFA salt of the title compound as colourless solid (0.100 g, 78%); ¹H NMR (400 MHz, MeOD) δ 7.84 - 7.78 (m, 1 H), 7.78 - 7.68 (m, 2 H), 7.50 - 7.44 (m, 2 H), 7.42 (s, 2 H), 7.33 (s, 1 H), 7.26 (d, *J* = 8.0, 1 H), 7.13 (t, *J* = 7.8, 1 H), 6.97 (d, *J* = 8.2, 1 H), 6.65 (d, *J* = 7.4, 1 H), 4.75 - 4.51 (m, 3 H), 4.38 (d, *J* = 12.0, 1 H), 3.76 - 3.63 (m, 1 H), 3.55 - 3.41 (m, 1 H), 3.35 - 3.24 (m, 1 H), 3.22 - 3.12 (m, 1 H), 3.12 - 3.01 (m, 1 H), 2.90 (t, *J* = 11.4, 1 H), 2.85 - 2.73 (m, 1 H), 2.73 - 2.62 (m, 1 H), 2.09 (s, 3 H), 1.84 - 1.69 (m, 1 H), 1.50 - 1.40 (m, 1 H); ¹³C NMR (101 MHz, MeOD) δ 171.76, 140.08, 139.93, 135.63, 134.56, 134.50, 130.18, 129.39, 128.93, 128.69, 128.03, 127.35, 127.30, 126.59, 126.08, 122.41, 120.17, 76.71, 73.16, 50.75, 47.15, 44.23, 37.69, 37.22, 27.42, 23.76; HRMS (ESI): *m*/*z* [M + H]⁺ calcd for C₂₈H₃₂N₅O₂: 470.2556; found 470.2553.
1. Alvarez, S. G.; Alvarez, M. T. Synthesis 1997, 1997, 413.
2. Barr, L.; Lincoln, S. F.; Easton, C. J. Supramolecular Chemistry 2005, 17, 547.
3. Harmsen, R. A. G.; Sydnes, L. K.; Törnroos, K. W.; Haug, B. E. Synthesis 2011, 5, 749.

### Example 1

The compounds shown in figure 2 have been tested for their inhibitory activity against human renin using the SensoLyte® Renin Assy Kit purchased from BioSite, Sweden for screening of renin inhibitors using an 5-FAM/QXL™520 fluorescence resonance energy transfer (FRET) peptide following the manufacturer's instructions. The sequence of the FRET probe is derived from the cleavage site of renin and the fluorescence of 5-FAM is quenched by OXL™520. Renin cleaves the FRET probe into two fragments recovering the fluorescence of 5-FAM that was monitored at 490/520 nm (excitation/emission).

In brief, all kit components were thawed to room temperature before starting the experiments. Renin substrate solution, Renin diluent and Renin inhibitor were prepared according to manufacturer's instructions by diluting the renin substrate (the FRET probe) and the inhibitor solution each 1 :100 and the renin 1 :80 in assay buffer. Test compounds (10 µl each) and renin diluent solution (40 µl) were added into 96-well plate wells. The following controls were set up at the same time (using assay buffer the total volume of all controls was brought up to 50 µl): positive control (diluted renin without test compound), inhibitor control (diluted renin and diluted renin inhibitor), test compound control (assay buffer and test compound) and substrate control (assay buffer). The plates were pre-incubated at 37°C for 30 min. At the same time, the renin substrate solution was pre-incubated at 37°C. For starting the enzymatic reaction 50 µl of renin substrate was added into each well. The reagents were mixed completely by shaking the plate gently for not more than 30 seconds. End-point reading of the enzymatic reaction was performed as follows: The reaction was incubated at 37°C for 15 min while the plate was kept out of direct light. The fluorescence intensity was measured at Ex/Em=490nm/520nm. The fluorescence readings were expressed in relative fluorescence units (RFU). The fluorescence reading from the substrate control expressed the background fluorescence and was substracted from the readings of the other wells. The RFU was plotted versus the concentration of the test compounds. The remaining protease activity was determined after incubation with the different triazolylpiperidine derivatives according to the present invention at different concentrations as identified in figure 1. Figure 1 shows that compound 37 and 39 are the most strong inhibitors of human renin among the tested compounds (remaining protease activity of 1.3 at 75 µM triazolylpiperidine derivative 37 concentration).

## Claims

1. A triazolylpiperidine compound of general formula III or IV 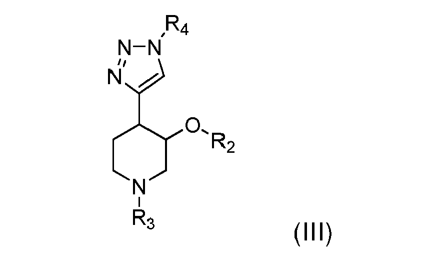 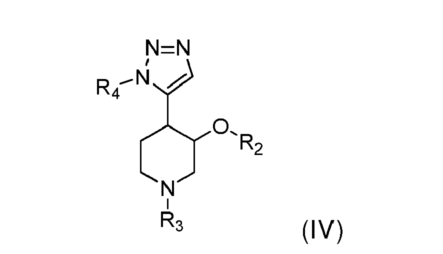 wherein R₂ and R₃ are independently from each other hydrogen or a C₁ - C₁₈ alkyl group, a C₂ - C₁₈ alkenyl group, a C₃ - C₁₈ aryl group, a C₃ - C₁₈ heteroalkyl group, a C₃ - C₁₈ heteroaryl group, an aralkyl group, an arylalkyl group, an aryl group, an heteroarylalkyl group, an alkoxy group, an acyl group, like a carbonylalkyl group or a carbonylaryl group, all of said groups may be substituted with at least one substituent independently selected from the group of C₁-C₆ alkyl, C₂-C₆ alkenyl, halogen, CN, NO₂, C₁-C₆ alkoxy, hydroxyl, carbamoyl, C₁ to C₈ alkylcarbamoyl, amido, C₁-C₆ alkoxycarbonyl, C₁-C₆ alkylcarbamoyloxy, C₁-C₆ alkoxycarbonylamino, amino, C₁-C₆ alkanoyloxy group, C₁-C₆ alkylthio and sulphur-containing analogs of oxygen containing substituents;
R4 is selected from hydrogen or a C1 -C18 alkyl group, a C2 - C18 alkenyl group, a C3 - C18 aryl group, a C3 - C18 heteroalkyl group, a C3 - C18 heteroaryl group, an aralkyl group, an arylalkyl group, an aryl group, an heteroarylalkyl group, an alkoxy group, an acyl group, like a carbonylalkyl group or a carbonylaryl group, all of said groups may be substituted with at least one substituent independently selected from the group of C1-C6 alkyl, C2-C6 alkenyl, halogen, CN, NO2, C1-C6 alkoxy, hydroxyl, carbamoyl, C1 to C8 alkylcarbamoyl, amido, *N-*substituted amido, C1-C6 alkoxycarbonyl, C1-C6 alkylcarbamoyloxy, C1-C6 alkoxycarbonylamino, amino, C1-C6 alkanoyloxy group, C1-C6 alkylthio and sulphur-containing analogs of oxygen containing substituents, or a substituent of formula V
- X - Y - Z (V)
wherein X and Z are independently selected from a C₁ - C₁₈ alkyl group, a C₂ - C₁₈ alkenyl group, a C₃ - C₁₈ aryl group, a C₃ - C₁₈ heteroalkyl group, a C₃ - C₁₈ heteroaryl group, an aralkyl group, an arylalkyl group,
an aryl group, an heteroarylalkyl group, an alkoxy group, an acyl group, like a carbonylalkyl group or a carbonylaryl group, all of said groups may be substituted with at least one substituent independently selected from the group of C₁-C₆ alkyl, C₂-C₆ alkenyl, halogen, CN, NO₂, C₁-C₆ alkoxy, hydroxyl, carbamoyl, C₁ to C₈ alkylcarbamoyl, amido, C₁-C₆ alkoxycarbonyl, C₁-C₆ alkylcarbamoyloxy, C₁-C₆ alkoxycarbonylamino, amino, C₁-C₆ alkanoyloxy group, C₁-C₆ alkylthio and sulphur-containing analogs of oxygen containing substituents; Y is selected from an amide group, like an acetamide group, carbonyl group, carboxyl group, carbamoyl, alkoxy, amino, whereby Y and/or Z may be absent; or salts thereof, for use in the prophylaxis or treatment of hypertension and diseases, disorders or conditions due to or involving hypertension or high blood pressure.

2. The compound of general formula III or IV as defined in claim 1 for inhibiting the aspartic protease rennin.

3. The compounds of general formula III or IV according to claim 1 or 2 wherein R₃ is hydrogen.

4. The compound according to any one of claims 1 to 3 wherein the substituents at position 3 and position 4 of the piperidine ring are in trans position.

5. The compounds according to anyone of the preceding claims wherein R₂ is selected form hydrogen, C₁ to C₈ alkyl, an arylalkyl group, an arylketo group, an aryl group.

6. The compounds according to any one of the preceding claims wherein R₄ is selected from a substituent of formula V
-X-Y-Z (V)
wherein X and Z are independently selected from a C₁ - C₁₈ alkyl group, a C₂ - C₁₈ alkenyl group, a C₃ - C₁₈ aryl group, a C₃ - C₁₈ heteroalkyl group, a C₃ - C₁₈ heteroaryl group, an aralkyl group, an arylalkyl group, an aryl group, an heteroarylalkyl group, an alkoxy group, an acyl group, like a carbonylalkyl group or a carbonylaryl group, all of said groups may be substituted with at least one substituent independently selected from the group of C₁-C₆ alkyl, C₂-C₆ alkenyl, halogen, CN, NO₂, C₁-C₆ alkoxy, hydroxyl, carbamoyl, C₁ to C₈ alkylcarbamoyl, amido, C₁-C₆ alkoxycarbonyl, C₁-C₆ alkylcarbamoyloxy, C₁-C₆ alkoxycarbonylamino, amino, C₁-C₆ alkanoyloxy group, C₁-C₆ alkylthio and sulphur-containing analogs of oxygen containing substituents; Y is selected from an amide group, like an acetamide group, carbonyl group, carboxyl group, carbamoyl, alkoxy, amino, whereby Y and/or Z may be absent.

7. The compounds according to anyone of the preceding claims wherein R₂ is an acetonaphtone group or a naphtylmethyl group, which may optionally be substituted as defined above.

8. The compounds according to any one of the preceding claims wherein R₄ is selected from a phenyl group, benzyl group, phenethyl group whereby said groups may be substituted with an alkoxy group, in particular, a methoxy group at position 4 of the aromatic ring, an acetamido group, an *N-*(naphthalene-2-ylmethyl)acetamido group, particularly an *N-*(naphthalene-2-ylmethyl)acetamido group at position 4 of the aromatic ring.

9. The compound according to any one of the preceding claims which is selected from:
(±)-*trans*-3-(Naphthalene-2-ylmethoxy)-4-(1-phenethyl-1*H*-1,2,3-triazol-4-yl)piperidine;
(±)-*trans*-3-Methoxy-4-(1-phenethyl-1*H*-1,2,3-triazol-4-yl)piperidine;
(±)-*trans*-3-(Benzyloxy)-4-(1-phenethyl-1*H*-1,2,3-triazol-4-yl)piperidine;
(±)-*trans*-3-Hydroxy-4-(1-(4-methoxy-phenethyl)-1*H*-1 ,2,3-triazol-4-yl)piperidine;
(±)-*trans*-4-(1-(4-Methoxyphenethyl)-1*H*-1,2,3-triazol-4-yl)-3-(naphthalen-2-ylmethoxy)piperidine;
(±)-*trans*-3-Methoxy-4-(1-(4-methoxy-phenethyl)-1*H*-1,2,3-triazol-4-yl)piperidine;
(±)-*trans*-3-Benzyloxy-4-(1-(4-methoxy-phenethyl)-1*H*-1,2,3-triazol-4-yl)piperidine;
(±)-*trans*-3-(Naphthalen-2-ylmethoxy)-4-(1-phenyl-1*H*-1,2,3-triazol-4-yl)piperidine;
(±) *trans*-*N*-(4-(2-(4-(3-(Naphthalen-2-ylmethoxy)piperidin-4-yl)-1*H*-1,2,3-triazol-1-yl) ethyl)phenyl) )-*N*-(naphthalene-2-ylmethyl)acetamide;
(±) *trans*-*N*-(3-(2-(4-(3-(Naphthalen-2-ylmethoxy)piperidin-4-yl)-1*H*-1,2,3-triazol-1-yl) ethyl)phenyl)-*N*-(naphthalene-2-ylmethyl)acetamide;
(±)-*trans*-3-(Naphthalen-2-ylmethoxy)-4-(1-phenethyl-1*H*-1,2,3-triazol-5-yl)piperidine;
(±)-*trans*-4-(1-(4-Methoxyphenethyl)-1*H*-1,2,3-triazol-5-yl)-3-(naphthalen-2-ylmethoxy)piperidine;
(±) *trans*-*N*-(4-(2-(5-(-3-(Naphthalen-2-ylmethoxy)piperidin-4-yl)-1*H*-1,2,3-triazol-1-yl)ethyl)phenyl)-*N*-(naphthalen-2-ylmethyl)acetamide;
(±)-*trans*-4-(1-(4-Methoxyphenethyl)-1*H*-1,2,3-triazol-4-yl)piperidin-3-yl 2-naphthoate;
(±)-*trans*-4-(1-(4-Methoxyphenethyl)-1*H*-1,2,3-triazol-4-yl)piperidin-3-yl 2-benzoate;
(±)-*trans*-4-(1-phenyl-1*H*-1,2,3-triazol-4-yl)piperidin-3-yl 2-naphthoate;
(±)-*trans*-4-(1-phenyl-1*H*-1,2,3-triazol-4-yl)piperidin-3-yl 2-benzoate.

10. The compound according to any one of the preceding claims wherein the compound is the (3R, 4R) enantiomer.

11. Pharmaceutical composition containing a compound according to any one of claims 1 to 10.

12. The pharmaceutical composition for use in the prophylaxis or treatment of hypertension and diseases, disorders or conditions due to or involving hypertension or high blood pressure.
